# EUROPEAN PATENT SPECIFICATION

(11) **EP 2 061 448 B1**
(45) Date of publication and mention of the grant of the patent: **17.07.2019**
(21) Application number: 07795652.2
(22) Date of filing: 31.05.2007
(51) Int. Cl.: A61K 31/135, A61K 31/137, A61K 31/485, A61K 9/20

(54) **SUSTAINED RELEASE FORMULATION OF NALTREXONE**
NALTREXON-FORMULIERUNG MIT VERZÖGERTER FREISETZUNG
FORMULATION À LIBÉRATION PROLONGÉE DE NALTRÉXONE

(30) Priority: 05.06.2006 US 811251 P; 29.08.2006 US 841114 P
(43) Date of publication of application: 27.05.2009
(73) Proprietor: Nalpropion Pharmaceuticals, Inc., Morristown, NJ 07960 (US)
(72) Inventor: MCKINNEY, Anthony A., San Diego, California 92130 (US); TOLLEFSON, Gary D., Indianapolis, Indiana 46236 (US); SOLTERO, Richard, Holly Springs, North Carolina 27540 (US); DUNZO, Thea Elise, Durham, North Carolina 27713 (US)
(74) Representative: Chapman, Desmond Mark
(86) International application number: PCT/US2007/013030
(87) International publication number: WO 2007/145863

(56) References cited:
- WO-A-83/03197
- WO-A-2004/071423
- WO-A-2005/032555
- WO-A-2007/067341
- WO-A-2007/089318
- US-A1- 2002 198 227
- US-A1- 2003 068 371
- US-A1- 2004 254 208
- US-B1- 6 306 436

## Description

### Field of the Invention

The present invention relates to pharmaceutical compositions as defined in the claims comprising sustained-release opioid receptor antagonists.

### Description of the Related Art

WO 2004/071423 concerns a method of treating pain in a patient comprising orally administering an opioid antagonist.

WO 83/03197 concerns a method for controlling gastrointestinal dysmotility in humans by administration of opioid antagonists.

US 2003/068371 concerns an oral dosage form comprising a therapeutically effective amount of an opioid analgesic, an opioid antagonist and one or more pharmaceutically acceptable excipients.

US 2002/198227 concerns a method for curbing dietary cravings in a patient, typically a diabetic or obese patient, by administration of a low dose of naltrexone to the patient.

WO2005/032555 concerns a pharmaceutical composition comprising from about 5 to about 20 mg of hydrocodone or a pharmaceutically acceptable salt thereof and from 0.055 to about 0.56 mg naltrexone or pharmaceutically acceptable salt thereof.

US 6306436 concerns stabilized bupropion hydrochloride pharmaceutical compositions.

Obesity is a major health concern in Western societies. It is estimated that about 97 million adults in the United States are overweight or obese. Epidemiological studies have shown that increasing degrees of overweight and obesity are important predictors of decreased life expectancy. Obesity causes or exacerbates many health problems, both independently and in association with other diseases. The medical problems associated with obesity, which can be serious and life-threatening, include hypertension; type 2 diabetes mellitus; elevated plasma insulin concentrations; insulin resistance; dyslipidemias; hyperlipidemia; endometrial, breast, prostate and colon cancer; osteoarthritis; respiratory complications, such as obstructive sleep apnea; cholelithiasis; gallstones; arterioscelerosis; heart disease; abnormal heart rhythms; and heart arrythmias (Kopelman, P. G., Nature 404, 635-643 (2000)). Obesity is further associated with premature death and with a significant increase in mortality and morbidity from stroke, myocardial infarction, congestive heart failure, coronary heart disease, and sudden death.

Obesity is often treated by encouraging patients to lose weight by reducing their food intake or by increasing their exercise level and therefore increasing their energy output. A sustained weight loss of 5% to 10% of body weight has been shown to improve the co-morbidities associated with obesity, such as diabetes and hypertension, and can lead to improvement of obesity-related conditions such as osteoarthritis, sleep apnea and pulmonary and cardiac dysfunction.

Naltrexone, having the chemical name (17-(cyclopropylmethyl)-4,5α-epoxy-3,14-dihydroxymorphinan-6-one) and the chemical structure shown below, is an opioid receptor antagonist used primarily in the management of alcohol dependence and opioid dependence.

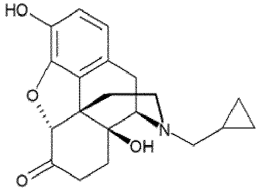

Naltrexone for oral administration has been commercially available for a number of years from various sources as the hydrochloride salt, naltrexone hydrochloride, e.g., under the trade names REVIA™ (50 mg) and DEPADE™ (25 mg, 50 mg and 100 mg). The currently approved forms of oral naltrexone are immediate release formulations that are efficacious even when dosed as infrequently as once every 72 hours. For example, the label of the DEPADE® brand of naltrexone hydrochloride indicates that naltrexone is a potent opioid antagonist with a prolonged pharmacological effect (24 to 72 hours) and recommends a dose of 50 mg once daily. The DEPADE® label discloses that clinical studies indicate that 50 mg of naltrexone hydrochloride will block the pharmacologic effects of 25 mg of intravenously administered heroin for periods as long as 24 hours. The DEPADE® label goes on to indicate that other data suggest that doubling the dose of naltrexone hydrochloride provides blockade for 48 hours, and tripling the dose of naltrexone hydrochloride provides blockade for about 72 hours. Thus, despite reaching a peak serum concentration quickly (Tₘₐₓ of approximately 1 hour) after oral administration, the immediate release form of naltrexone has relatively long lasting effects.

The long-lasting effects of the immediate release form of oral naltrexone may be used to encourage patient compliance by utilizing a dosing frequency that is less than once per day, e.g. every other day or every three days. For example, the DEPADE® label indicates that a flexible approach to a dosing regimen may be employed to enhance compliance. Thus, the DEPADE® label discloses, patients may receive 50 mg of naltrexone hydrochloride every weekday with a 100 mg dose on Saturday or patients may receive 100 mg every other day, or 150 mg every third day. The DEPADE® label refers to clinical studies reported in the literature that have employed the following dosing regimen: 100 mg on Monday, 100 mg on Wednesday, and 150 mg on Friday. Thus, use of the immediate release oral form allows a patient to take a relatively large dose of naltrexone at a time when the temptation to abuse alcohol or opioids may be less (e.g., during the week), with the effects lasting to a time when temptation may be greater (e.g., over the weekend).

The DEPADE® label indicates that naltrexone has not been shown to cause significant increases in complaints in placebo-controlled trials in patients known to be free of opioids for more than 7 to 10 days. Although a subset of the patient population reports nausea upon initial administration of 25 mg or 50 mg dosages of the immediate release oral form of naltrexone, the nausea often subsides as those patients develop a tolerance. The DEPADE® label indicates that, in an open label safety study with approximately 570 individuals with alcoholism receiving naltrexone, the following new-onset adverse reactions occurred in 2% or more of the patients: nausea (10%), headache (7%), dizziness (4%), nervousness (4%), fatigue (4%), insomnia (3%), vomiting (3%), anxiety (2%) and somnolence (2%). Moderate to severe nausea was reported by 18 patients (15%) in a 10-week open label study of naltrexone among alcohol dependent participants, involving a initial 25 mg dose followed by a dose of 50 mg daily for 10 weeks (O'Malley, S.S. J. Clin. Psychopharmacol. 2000 Feb;20(1):69-76). Eight of the eighteen patients that experienced moderate to severe nausea discontinued treatment, nausea resolved within one week for five subjects and within two weeks for four, and it continued off and on throughout the ten-week period for one subject. The authors linked the moderate to severe nausea to poorer medication compliance and heavier drinking by the patients during treatment.

It appears that while some patients experience adverse effects upon administration of 25 mg or 50 mg dosages of immediate release oral naltrexone, the adverse effects often subside within a relatively short period of time and many patients are able to tolerate significantly higher oral dosages, e.g., 100 mg or 150 mg. A once-monthly injectable form of naltrexone is commercially available under the tradename VIVITROL® for the treatment of alcoholism. The prescribing information for VIVITROL® indicates that patients should be advised that they may experience nausea following the initial injection of the VIVITROL® naltrexone; that these episodes of nausea tend to be mild and subside within a few days post-injection, and that patients are less likely to experience nausea in subsequent injections.

U.S. Patent Publication No. 2004/0254208 A1 discloses the use of naltrexone in combination with other compounds for affecting weight loss, but does not disclose any particular adverse effects associated with the combinations.

### SUMMARY OF THE INVENTION

The invention provides a dosage form comprising a sustained-release formulation of naltrexone, or a pharmaceutically acceptable salt thereof and a sustained-release formulation of bupropion or a pharmaceutically acceptable salt thereof, wherein the dosage form is an oral dosage form, wherein the dosage form provides an in vitro release rate of the naltrexone of less than 80% or less than 70% in about 1 hour, of less than 90% or less than 80% in about 2 hours, or of less than 98% in about 4 hours, as determined using United States Pharmacopeia 24th edition (USP 24) Apparatus 2 dissolution test having a spindle rotation speed of 100 rpm and a dissolution medium of water, at 37°C.

An unexpectedly high incidence of adverse effects associated with co-administration of naltrexone with bupropion and/or fluoxetine has now been identified as a problem. For example, during a clinical trial described in greater detail below, the incidence of adverse events of moderate severity associated with co-administration of immediate-release naltrexone with bupropion was 30.7%. This incidence of adverse effects was significantly higher than would be expected based on the typical incidence of adverse effects associated with administration of immediate-release naltrexone alone or bupropion alone.

In an embodiment, sustained release naltrexone formulations as defined in the claims have now been developed that provide a solution to this problem.

An embodiment provides an oral dosage form as defined in the claims, comprising a sustained-release naltrexone formulation that is formulated to provide a reduction in at least one adverse effect, wherein the adverse effect is associated with co-administration of an immediate-release naltrexone formulation and a second compound which is a sustained release formulation of the monoamine reuptake inhibitor bupropion. Also described herein is a method of administering naltrexone, comprising administering a sustained-release naltrexone formulation to a subject, e.g., in a manner that is effective to cause weight loss and/or inhibit weight gain.

Another embodiment provides an oral unit dosage form as defined in the claims, comprising a sustained-release naltrexone formulation that is effective to provide, after administration, an in vivo plasma concentration profile comprising at least one selected from:
(a) a naltrexone Cₘₐₓ that is about 80% or less of the naltrexone Cₘₐₓ of REVIA™ immediate-release naltrexone hydrochloride, and a naltrexone AUCₗₐₛₜ that is in the range of about 80% to about 125% of the naltrexone AUCₗₐₛₜ of REVIA™ immediate-release naltrexone hydrochloride; and
(b) a 6-beta naltrexol Cₘₐₓ that is about 80% or less of the 6-beta naltrexol Cₘₐₓ of REVIA™ immediate-release naltrexone hydrochloride, and a 6-beta naltrexol AUCₗₐₛₜ that is in the range of about 80% to about 125% of the 6-beta naltrexol AUCₗₐₛₜ of REVIA™ immediate-release naltrexone hydrochloride.

Another embodiment provides an oral dosage form as defined in the claims, comprising naltrexone or a pharmaceutically acceptable salt thereof and a sustained-release carrier composition, wherein the oral dosage form provides an in vitro release rate of the naltrexone or pharmaceutically acceptable salt thereof of less than about 90% in about 4 hours.

Also described herein is a method of administering a sustained-release naltrexone formulation as described herein, for example, a method of administering naltrexone, comprising administering a sustained-release naltrexone formulation to a subject in an amount that is effective to provide an in vivo plasma concentration profile comprising at least one selected from:
(a) a naltrexone Cₘₐₓ that is about 80% or less of the naltrexone Cₘₐₓ of REVIA™ immediate-release naltrexone hydrochloride, and a naltrexone AUCₗₐₛₜ that is in the range of about 80% to about 125% of the naltrexone AUCₗₐₛₜ of REVIA™ immediate-release naltrexone hydrochloride; and
(b) a 6-beta naltrexol Cₘₐₓ that is about 80% or less of the 6-beta naltrexol Cₘₐₓ of REVIA™ immediate-release naltrexone hydrochloride, and a 6-beta naltrexol AUCₗₐₛₜ that is in the range of about 80% to about 125% of the 6-beta naltrexol AUCₗₐₛₜ of REVIA™ immediate-release naltrexone hydrochloride.

In an embodiment, a dosage form as described in the claims is for use in treating a weight related condition, e.g., to cause weight loss and/or inhibit weight gain.

These and other embodiments are described in greater detail below.

### BRIEF DESCRIPTION OF THE DRAWINGS

Figure 1 shows the dissolution profile of sustained release 5 mg naltrexone tablets containing polyethylene oxide.
Figure 2 shows the dissolution profile of sustained release 5 mg naltrexone tablets containing hydroxypropylmethyl cellulose.
Figure 3 shows the dissolution profile of sustained release naltrexone and bupropion tablets containing hydroxypropylmethyl cellulose.
Figure 4 shows the individual plasma concentration time curves of naltrexone in subjects receiving Naltrexone IR
Figure 5 shows the individual plasma concentration time curves of naltrexone in subjects receiving Naltrexone SR.
Figure 6 shows the individual plasma concentration time curves of 6-beta naltrexol in subjects receiving Naltrexone IR.
Figure 7 shows the individual plasma concentration time curves of 6-beta naltrexol in subjects receiving Naltrexone SR.
Figures 8A and 8B show the average plasma concentration time curves of naltrexone across subjects receiving Naltrexone SR (circles) or Naltrexone IR (squares) across two time scales.
Figures 9A and 9B show the average plasma concentration time curves of 6-beta naltrexol across subjects receiving Naltrexone SR (circles) or Naltrexone IR (squares) across two time scales.
Figure 10 is a schematic illustrating the population of subjects reporting nausea and vomiting using the UKU Adverse Event Rating Scale.
Figure 11 shows the average plasma concentrations of naltrexone in subjects receiving naltrexone IR in combination with bupropion SR (●) or naltrexone SR in combination with bupropion SR (■).
Figure 12 shows the average plasma concentrations of 6-beta naltrexol in subjects receiving naltrexone IR in combination with bupropion SR (●) or naltrexone SR in combination with bupropion SR (■).
Figure 13 shows the average trough plasma concentrations of naltrexone in subjects receiving naltrexone IR in combination with bupropion SR (●) or naltrexone SR in combination with bupropion SR (■).
Figure 14 shows the average trough plasma concentrations of 6-beta naltrexol in subjects receiving naltrexone IR in combination with bupropion SR (●) or naltrexone SR in combination with bupropion SR (■).
Figure 15 shows the average plasma concentrations of bupropion in subjects receiving bupropion SR in combination with naltrexone SR (●) or in combination with naltrexone IR (■).
Figure 16 shows the average plasma concentrations of the bupropion metabolite hydroxybupropion in subjects receiving bupropion SR in combination with naltrexone SR (●) or in combination with naltrexone IR (■).
Figure 17 shows the average plasma concentrations of the bupropion metabolite threohydroxybupropion in subjects receiving bupropion SR in combination with naltrexone SR (●) or in combination with naltrexone IR (■).
Figure 18 shows the average plasma concentrations of the bupropion metabolite erythrohydroxybupropion in subjects receiving bupropion SR in combination with naltrexone SR (●) or in combination with naltrexone IR (■).

### DETAILED DESCRIPTION OF THE PREFERRED EMBODIMENTS

Various embodiments provide an oral dosage form as defined in the claims that comprises a sustained-release naltrexone formulation. Co-administration of a sustained-release naltrexone formulation with sustained-release bupropion may provide a reduction in at least one adverse effect as compared to co-administration of an immediate-release naltrexone formulation with sustained-release bupropion. The at least one adverse effect may include nausea.

The oral dosage form may provide a naltrexone Cₘₐₓ and/or a 6-beta naltrexol Cₘₐₓ that is about 80% or less of the naltrexone Cₘₐₓ and/or the 6-beta naltrexol Cₘₐₓ of REVIA™ immediate-release naltrexone hydrochloride. The oral dosage form may provide a naltrexone AUCₗₐₛₜ and/or a 6-beta naltrexol AUCₗₐₛₜ that is in the range of about 80% to about 125% of the naltrexone AUCₗₐₛₜ and/or the 6-beta naltrexol AUCₗₐₛₜ of REVIA™ immediate-release naltrexone hydrochloride.

### Definitions

The term "naltrexone" may be used in a general way herein to refer to a free base of naltrexone, a pharmaceutically acceptable naltrexone salt (including hydrates and anhydrous forms, e.g., naltrexone hydrochloride dihydrate and anhydrous naltrexone hydrochloride), a naltrexone metabolite, a naltrexone isomer, a naltrexone prodrug or mixtures thereof. Reference herein to "naltrexone" will be understood as encompassing all such forms, unless the context clearly indicates otherwise.
The term "pharmaceutically acceptable salt" refers to a formulation of a compound that does not cause significant irritation to an organism to which it is administered and does not abrogate the biological activity and properties of the compound. Pharmaceutical salts of basic compounds can be obtained by reacting the compound with an acid such as hydrochloric acid, hydrobromic acid, sulfuric acid, nitric acid, phosphoric acid, methanesulfonic acid, ethanesulfonic acid, p-toluenesulfonic acid, salicylic acid and the like. For example, naltrexone hydrochloride is a pharmaceutically acceptable salt of naltrexone. Pharmaceutical salts of acidic compounds can be obtained by reacting the compound with a base to form a salt such as an ammonium salt, an alkali metal salt, such as a sodium or a potassium salt, an alkaline earth metal salt, such as a calcium or a magnesium salt, a salt of organic bases such as dicyclohexylamine, N-methyl-D-glucamine, tris(hydroxymethyl) methylamine, and salts thereof with amino acids such as arginine, lysine, and the like.
The term "oral dosage form", as used herein, has its ordinary meaning as understood by those skilled in the art and thus includes, by way of non-limiting example, a formulation of a drug or drugs in a form administrable to a human. The illustrative embodiments of the invention have been described primarily as being directed to oral dosage forms such as tablets, cores, capsules, caplets and loose powder, but other suitable dosage forms such as solutions and suspensions are also contemplated.
The term "sustained release", as used herein, has its ordinary meaning as understood by those skilled in the art and thus includes, by way of non-limiting example, the controlled release of a drug from a dosage form over an extended period of time. For example, in some embodiments, sustained-release dosage forms are those that have a release rate that is substantially longer than that of a comparable immediate release form, e.g., greater than 125% of the release rate of an immediate-release dosage form.
The term "immediate release", as used herein, has its ordinary meaning as understood by those skilled in the art and thus includes, by way of non-limiting example, release of a drug from a dosage form in a relatively brief period of time after administration. Examples of immediate-release dosage forms of naltrexone hydrochloride include REVIA™ immediate-release naltrexone hydrochloride and DEPADE™ immediate-release naltrexone hydrochloride. In some embodiments, immediate-release dosage forms are those that have a release rate that is up to and including 125% of the release rate for one or more of REVIA™ immediate-release naltrexone hydrochloride and DEPADE™ immediate-release naltrexone hydrochloride. The REVIA™ immediate-release naltrexone hydrochloride may be a 50 mg dosage form. The DEPADE™ immediate-release naltrexone hydrochloride may be a 25 mg, 50 mg or 100 mg dosage form. As used herein, an immediate-release formulation may refer to REVIA™ immediate-release naltrexone hydrochloride. In some embodiments, the dosage of the immediate-release formulation is substantially the same as a sustained-release dosage form described herein, while in other embodiments it is not.
The term "release rate", as used herein, has its ordinary meaning as understood by those skilled in the art and thus includes, by way of non-limiting example, a characteristic related to the amount of an active ingredient released per unit time as defined by in vitro or in vivo testing. An in vitro release rate is determined by a "standard dissolution test.," conducted according to United States Pharmacopeia 24th edition (2000) (USP 24), pp. 1941-1943, using Apparatus 2 described therein at a spindle rotation speed of 100 rpm and a dissolution medium of water, at 37°C, or other test conditions substantially equivalent thereto.
The term "pharmacokinetic profile," as used herein, has its ordinary meaning as understood by those skilled in the art and thus includes, by way of non-limiting example, a characteristic of the curve that results from plotting blood serum concentration of a drug over time, following administration of the drug to a subject. A pharmacokinetic profile thus includes a pharmacokinetic parameter or set of parameters that can be used to characterize the pharmacokinetics of a particular drug or dosage form when administered to a suitable patient population. Various pharmacokinetic parameters are known to those skilled in the art, including area under the blood plasma concentration vs. time curve (AUC) and maximum blood plasma concentration after administration (Cₘₐₓ). AUCₗₐₛₜ indicates the area under the blood plasma concentration vs. time curve from the time of administration until the time of the last measurable concentration. Pharmacokinetic parameters may be measured in various ways known to those skilled in the art, e.g., single dosage or steady-state. Differences in one or more of the pharmacokinetic profiles (e.g., Cₘₐₓ) may indicate pharmacokinetic distinctness between two formulations.

Those skilled in the art will understand that pharmacokinetic parameters may be determined by comparison to a reference standard using clinical trial methods known and accepted by those skilled in the art, e.g., as described in the examples set forth herein. Since the pharmacokinetics of a drug can vary from patient to patient, such clinical trials generally involve multiple patients and appropriate statistical analyses of the resulting data (typically ANOVA at 90% confidence). Comparisons of pharmacokinetic parameters are on a dose-adjusted basis, as understood by those skilled in the art.

In various embodiments related to the sustained-release naltrexone formulations described herein, the reference standard is an immediate-release naltrexone formulation. Those skilled in the art will understand that an immediate-release naltrexone formulation appropriate for use as the reference standard in the determination of pharmacokinetic parameters is the legend immediate-release naltrexone formulation, widely available commercially as the REVIA® brand of naltrexone hydrochloride, or an immediate-release naltrexone formulation that is formulated to have a pharmacokinetic profile that is substantially similar to the REVIA® brand of naltrexone hydrochloride. The U.S. government regulates the manner in which prescription drugs can be labeled and thus reference herein to the REVIA® brand of naltrexone hydrochloride has a well-known, fixed and definite meaning to those skilled in the art.

### Co-Administration of Naltrexone and a Second Compound

In some embodiments, it is recognized that co-administration of immediate-release naltrexone with a second compound as defined in the claims may be associated with at least one adverse effect. The at least one adverse effect may be of greater severity, duration and/or probability of occurrence than expected by separate administration of the immediate-release naltrexone and of the second compound as defined in the claims. In some embodiments, it is thought that the second compound as defined in the claims can potentiate naltrexone-induced effects in the chemoreceptor trigger zone (CTZ) and/or the vomiting center, thereby potentiating an adverse effect associated with administration of naltrexone. While the adverse effect may generally be characterized as tolerable when immediate-release naltrexone is administered alone, the potentiated adverse effect may be characterized as less tolerable.

Co-administration of sustained-release naltrexone with the second compound as defined in the claims may provide a reduction in at least one adverse effect as compared to the co-administration of immediate-release naltrexone with the second compound. The reduction may include a decrease in the severity, duration and/or probability of occurrence of the at least one adverse effect. In some cases, e.g., involving individual patients, such reductions may be in comparison to the adverse effects that would be expected by one of skill in the art in view of the known side effects of a immediate release form, and thus it is not necessary that the patient actually experience side effects from the immediate release form in order to benefit from such reductions in adverse effects. While not wishing to be bound to any particular theory, the reduction in the at least one adverse effect may be a result of a lower peak blood concentration of naltrexone or a metabolite thereof associated with the administration of sustained-release naltrexone as compared to that associated with the administration of immediate-release naltrexone. A sustained-release form of naltrexone with dissolution over a longer period may provide reduced excitation, antagonism and/or inhibition to the chemoreceptor trigger zone and vomiting center (VC) in the area postrema of the brain. Further, sustained-release oral dosage forms may result in a lower concentration available to be taken up by stomach opioid receptors.

In some embodiments, the at least one adverse effect is primarily associated with administration of the second compound as defined in the claims. Co-administration of sustained-release naltrexone can reduce the at least one adverse effect. In other embodiments, the at least one adverse effect is primarily associated with administration of the immediate-release naltrexone. Sustained-release naltrexone can provide a reduction in the at least one adverse effect as compared to the immediate-release formulation, and the sustained-release naltrexone may then be co-administered with the second compound. In still other embodiments, the at least one adverse effect is primarily associated with the combination of the second compound and the immediate-release naltrexone. The type of adverse effect associated with the combination may be one that is also associated with the administration of naltrexone alone and/or by the administration of the second compound alone. The severity, duration and/or probability of occurrence of the adverse effect may be greater when immediate-release naltrexone is co-administered with the second compound than would be expected from the separate administrations of immediate-release naltrexone and of the second compound. Co-administration of sustained-release naltrexone and the second compound can provide a reduction in the at least one adverse effect as compared to the co-administration of immediate-release naltrexone and the second compound.

The immediate-release naltrexone may comprise REVIA™ immediate-release naltrexone hydrochloride. The dosage of naltrexone in the immediate-release naltrexone may be substantially similar to that in the sustained-release naltrexone. The immediate-release naltrexone is one that is substantially free of a sustained-release carrier composition.

The second compound may include an antidepressant. The second compound may include a monoamine reuptake inhibitor and/or a monoamine modulator. The monoamine may include norepinephrine, dopamine and/or serotonin. The second compound may include a dopamine reuptake inhibitor and/or a dopamine modulator. Dopamine modulation may be an effect of serotonin modulation. The second compound may include a selective serotonin reuptake inhibitor and/or a serotonin modulator. The second compound may include a compound that acts on the chemoreceptor trigger zone (CTZ) and/or the vomiting center in the lateral medullary reticular formation. The second is bupropion or a pharmaceutically acceptable salt thereof. The pharmaceutically acceptable salt may comprise a hydrochloride salt. The second compound is a sustained release formulation.

The sustained-release naltrexone may be provided in an oral dosage form. In some embodiments, the oral dosage form also includes the second compound, while in other embodiments, it does not.

Co-administration of immediate-release naltrexone or sustained-release naltrexone with the second compound, i.e. sustained release bupropion, may include administering an oral dosage form comprising both the naltrexone and the second compound. The co-administration may include administering the naltrexone and the second compound separately but at substantially the same time. The co-administration may include administering the naltrexone and the second compound at different times (e.g., at different dosing schedules), but such that blood plasma simultaneously includes naltrexone or a naltrexone metabolite concentrations above a first threshold and concentrations for the second compound or for metabolites of the second compound above a second threshold. These thresholds may be the minimum detectable level (e.g., zero). This latter co-administration may be appropriate, for example, if the in vivo Tₘₐₓ or the dissolution rate of the naltrexone formulation is substantially different from that of the second compound.

The at least one adverse effect may include an effect associated with the chemoreceptor trigger zone (CTZ) and/or the vomiting center. In some embodiments, the at least one adverse effect includes one or more of nausea, retching, dizziness, stomach upset, vertigo and vomiting. The at least one adverse effect may include one or more of anxiety, nervousness, headache, sleeping trouble, sneezing, nasal stuffiness, muscle pain, decreased sexual function or desire, blurred vision, thirst, ringing in the ears, weakness, tiredness, skin rash, confusion, mood changes, hallucinations, severe diarrhea, and breathing trouble.

The at least one adverse effect may be associated with a weight-loss treatment. The weight-loss treatment may comprise, for example, administration of one or more of an atypical antipsychotic, an antidepressant, a monoamine reuptake inhibitor, a monoamine modulator, a norepinephrine reuptake inhibitor, a dopamine reuptake inhibitor, and/or a nicotinic antagonist. The weight-loss treatment may comprise administration of bupropion or a pharmaceutically acceptable salt thereof and/or fluoxetine or a pharmaceutically acceptable salt thereof. The weight-loss treatment may comprise administration of naltrexone. In some embodiments, the sustained-release naltrexone dosage form results in the reduction of one or more adverse effects attributed, partially or wholly, to one or more non-naltrexone active ingredients of a weight-loss treatment. In some embodiments, a sustained-release naltrexone dosage form results in the reduction of one or more adverse effects attributed, partially or wholly, to a naltrexone active ingredients of a weight-loss treatment.

### Pharmacokinetic Profile of Sustained-Release Naltrexone Formulations

In some embodiments, a sustained-release naltrexone formulation as defined in the claims provides a pharmacokinetic profile that is pharmacokinetically distinct from an immediate-release naltrexone formulation (e.g., REVIA™ immediate-release naltrexone hydrochloride). The pharmacokinetic distinction may be due to a difference between the Cₘₐₓ values associated with the immediate-release and sustained-release formulations. The sustained-release naltrexone formulation may provide a naltrexone Cₘₐₓ and/or a 6-beta naltrexol Cₘₐₓ that is about 80% or less of the naltrexone Cₘₐₓ and/or the 6-beta naltrexol Cₘₐₓ of an immediate-release naltrexone formulation (e.g., REVIA™ immediate-release naltrexone hydrochloride).

In some embodiments, the sustained-release naltrexone formulation as defined in the claims provides a naltrexone AUCₗₐₛₜ and/or a 6-beta naltrexol AUCₗₐₛₜ that substantially similar to (e.g., between about 80% to about 125% of) a naltrexone AUCₗₐₛₜ and/or a 6-beta naltrexol AUCₗₐₛₜ of an immediate-release naltrexone formulation (e.g., REVIA™ immediate-release naltrexone hydrochloride).

In some embodiments, the naltrexone dosage of the immediate-release naltrexone formulation is substantially the same as the naltrexone dosage of the sustained-release naltrexone formulation. In some embodiments in which a Cₘₐₓ value differs between the immediate-release and sustained release formulations, the naltrexone dosage of the sustained-release naltrexone formulation is identified as one that provides a naltrexone AUCₗₐₛₜ and/or a 6-beta naltrexol AUCₗₐₛₜ that is substantially similar to (e.g., between about 80% to about 125% of) a naltrexone AUCₗₐₛₜ and/or a 6-beta naltrexol AUCₗₐₛₜ of the sustained-release naltrexone formulation

The pharmacokinetic profile may be related to an effectiveness and/or a reduced side effect produced by the dosage form. For example, a sustained-release naltrexone formulation as defined in the claims may produce substantially similar or improved efficacy as compared to an immediate-release naltrexone formulation due to a substantially similar naltrexone and/or 6-beta naltrexol AUCₗₐₛₜ. As another example, a sustained-release naltrexone formulation may provide a reduction in an adverse effect as compared to that of an immediate-release naltrexone formulation due to a lower naltrexone and/or 6-beta naltrexol Cₘₐₓ.

Pharmacokinetic profiles can be determined by analyzing the plasma of a patient population that has received sustained-release naltrexone oral dosage forms, and comparing them to a comparable patient population that has received an immediate-release formulation, using the appropriate clinical trial methodology and statistical analyses.

In some embodiments, the pharmacokinetic properties are after a single- drug dosage, while in others, they are at steady-state. For the single-dosage measurements, patients may be provided with a single dosage of a composition comprising the sustained-release naltrexone, and plasma specimens may be collected from the patient at different time periods relative to the administration of the composition to determine pharmacokinetic profiles. For the steady-state measurements, patients may be provided with a dosing regimen across a plurality of days comprising administering oral dosage forms comprising sustained-release naltrexone. Plasma specimens may then be collected from the patient at different time periods relative to a particular dosage during steady state. In some embodiments, the steady state can be determined by monitoring a plasma naltrexone and/or active naltrexone metabolite (e.g., 6 beta-naltrexol) concentration profiles at specific times of anticipated peak and trough blood levels relative to the administration of a dosage across hours and days and determining when the profile has reached steady state. For example, the in vivo plasma naltrexone and/or active naltrexone metabolite (e.g., 6 beta-naltrexol) concentration may be measured one hour after dosing across days, until the concentration no longer significantly varies from day to day. In other embodiments, the steady state may be estimated as a specific number of days after the dosing regimen began. For example, steady state may be estimated as six days after the dosing regimen began. In some embodiments, steady state is estimated after dosing over about five times the half-life of the drug.

Plasma may be analyzed using any appropriate method. In some embodiments, blood is collected from a patient. Any suitable amount of blood may be collected. Blood samples may then be centrifuged until separation of red cells from plasma occurs. In some embodiments, naltrexone and/or naltrexone metabolite analysis is performed according to the analytical method validation entitled "Validation of a High Performance Liquid Chromatographic Method Using Tandem Mass Spectrometry Lithium Heparinized Plasma".

In order to measure the pharmacokinetic parameters mentioned above, in vivo naltrexone and/or naltrexone metabolite (e.g., 6 beta-naltrexol) concentrations may be measured at various time intervals with respect to a naltrexone dosage. In some embodiments, these concentrations are measured at least 10 times within a 24 hour period.

In some embodiments, a patient population undergoes a dosing regimen comprising the administration of a sustained-release naltrexone oral dosage form as described herein, and the reported pharmacokinetic parameters are the average of the pharmacokinetic parameters across patients. The average may be obtained by calculating the parameters for each patient and then averaging across patients. In some embodiments, the averaging comprises a least-squares arithmetic mean or a least-squares geometric mean. In some embodiments, pharmacokinetic parameters are obtained from crossover studies.

A sustained-release naltrexone formulation is provided and/or administered as an oral dosage form as defined in the claims. The oral dosage form may also include a second compound as defined in the claims. In an embodiment, oral dosage forms described herein are effective in the treatment of one or more of a weight-related condition. The weight-related condition may comprise a condition characterized by a body-mass index of greater than or equal to about 25 kg/m², greater than or equal to about 30 kg/m² or less than or equal to about 18.5 kg/m². The weight-related condition may comprise obesity. The weight-related condition may be caused or expected to be caused by administration of a medication. Oral dosage forms described herein may be effective in affecting (e.g., causing) weight loss, inhibiting weight gain and/or at least partially reversing weight gain.

The oral dosage form may be distributed, provided to a patent for self-administration or administered to a patient. The patient may be overweight or obese. The patient may have a body-mass index (BMI) of about 20 kg/m² or greater, about 25 kg/m² or greater, or about 30 kg/m² or greater. The patient may be obese. The patient may be suffering from diabetes.

Administration of sustained-release oral dosage forms described herein may provide substantially the same or better efficacy than immediate-release formulations. The sustained-release oral dosage forms may be associated with reduced adverse effects as compared to immediate-release formulations. In some embodiments, the administration of an oral dosage form described herein may result in improved patient compliance with a treatment (e.g., a weight-loss treatment and/or a naltrexone treatment).

In some embodiments, the present invention includes a dosage form as defined in the claims for use in a method of treatment, comprising identifying a patient suffering from at least one adverse side effect and/or who is particularly susceptible to at least one adverse side effect associated with a weight-loss treatment (e.g., a weight-loss treatment that comprises administration of bupropion) and/or treatment with an immediate-release naltrexone formulation (e.g., REVIA™ immediate-release naltrexone hydrochloride), and providing or administering to the patient a sustained-release naltrexone dosage form as described herein.

Such embodiments can include the step of administering a therapeutically-effective amount of the sustained-release dosage form to a mammal in need thereof by any suitable route or method of delivery, including those described herein. Actual dosage levels of the compounds in the pharmaceutical dosage forms may be varied so as to administer an amount of naltrexone that is effective to achieve the desired therapeutic response for a particular patient.

### Dosages

It will be understood that the specific dose level of the sustained-released dosage forms described herein for any particular patient can depend upon any of a variety of factors including the genetic makeup, body weight, general health, diet, time and route of administration, combination with other drugs and the particular condition being treated, and its severity. In some embodiments, the naltrexone dosage of the sustained-release dosage forms as described in the claims is at least partially determined based upon the frequency of which the oral dosage form is administered. Sustained-release oral dosage forms may be administered more frequently, less frequently, or at substantially the same frequency as immediate-release formulations (e.g., REVIA™ immediate-release naltrexone hydrochloride).

In some embodiments, a sustained-release oral dosage form as defined in the claims comprising naltrexone provides dosages in a naltrexone free base equivalent amount in the range of from about 4 mg to about 50 mg or in the range of about 10 mg to about 25 mg. The oral dosage form may include about 4 mg, about 8 mg, about 12 mg, about 16 mg, about 32 mg, or about 48 mg of naltrexone. The selection of a particular dosage may be based on the weight of the patient. The selection of a particular dosage may be based on the identity, dosage and/or dosing schedule of another compound being co-administered. Unit dosage forms suitable for administration to a human may be configured to provide a naltrexone free base equivalent dosage in the range of about 0.75 mg/kg to about 10 mg/kg, e.g., about 2 mg/kg (basis is mg of drug per kilogram of body weight). Dosages may be at least partially determined by a pharmacokinetic profile. For example, an amount of a sustained-release naltrexone oral dosage form may be administered that is sufficient to provide an AUCₗₐₛₜ substantially similar to that of an immediate-release naltrexone oral dosage form.

The sustained-release dosage forms as defined in the claims may be administered one, two or more times per day, with or without a loading dose. In some embodiments, the number of administrations per day is constant (e.g., one time per day). In other embodiments, it is variable. The number of administrations may change depending on effectiveness of the dosage form, observed side effects, external factors (e.g., a change in another medication), or the length of time that the dosage form has been administered.

In an embodiment, a sustained-release naltrexone dosage form as defined in the claims further comprises a second compound, as described herein. The second compound may be present in any appropriate dosage. The second compound comprises bupropion or a pharmaceutically acceptable salt thereof in a sustained release (SR) formulation. In an embodiment, the dosage form comprises both naltrexone and bupropion, e.g., in the form of a tri-layer tablet as described in U.S. Provisional Patent Application Serial Number 60/865,157, filed November 9, 2006. Examples of suitable unit dosage forms include those in which the tri-layer tablet includes, e.g., about 4 mg naltrexone SR and 90 mg of bupropion SR; about 8 mg naltrexone SR and 90 mg of bupropion SR; or about 12 mg naltrexone SR and 90 mg of bupropion SR. In an embodiment, the dosage form may be administered as two tablets twice a day, e.g., for a daily dosage of about 16 mg, about 32 mg, or about 48 mg naltrexone, and about 360 mg bupropion or a pharmaceutically acceptable salt thereof. In an embodiment, the second compound (i.e. sustained-release bupropion or a pharmaceutically acceptable salt thereof) and naltrexone are co-administered separately, e.g., as separate dosage forms that are co-administered within about an hour of each other. In an embodiment, the separate dosage forms are administered at about the same time.

### Formulations

Oral dosage forms as defined in the claims may comprise naltrexone and a sustained-release carrier. A sustained release carrier includes, by way of non-limiting example, an ingredient or ingredients that are included in a pharmaceutical formulation in amounts that are effective to extend the release rate of naltrexone from the formulation as compared to an immediate-release formulation (e.g., REVIA™ immediate-release naltrexone hydrochloride). A sustained release carrier may be referred to herein as a retardant excipient. Examples of sustained release carriers include hydroxypropylmethyl cellulose, polyethylene oxide, polyacrylate, copolymer of acrylate and methacrylate, methacrylate polymer, copolymer of acrylate and methacrylate, copolymer of acrylate and methacrylate with ammonium group, copolymer of maleic anhydride and methyl vinyl ether, hydroxy propyl ethyl cellulose, hydroxy propyl cellulose, hydroxy ethyl cellulose, methyl cellulose, hydroxymethyl methacrylate, maltodextrin, natural gum and xanthan gum. In some embodiments, the sustained-release carrier composition comprises at least one of hydroxypropylmethylcellulose and polyoxyethylene. A sustained release carrier composition may contain one or more sustained release carriers, along with other suitable ingredients.

In some embodiments, an oral dosage form as defined in the claims comprising naltrexone comprises an amount of a sustained-release carrier composition that is effective to render the dosage form pharmacokinetically distinct from an immediate-release formulation (e.g., REVIA™ immediate-release naltrexone hydrochloride). For example, relative to the immediate-release formulation, the amount and type of sustained-release carrier composition may be selected to reduce the naltrexone Cₘₐₓ and/or the 6-beta naltrexol Cₘₐₓ (e.g., to about 80% or less than the naltrexone Cₘₐₓ of or 6-beta naltrexol Cₘₐₓ of immediate-release naltrexone).

The amount of the sustained-release carrier composition may be effective to provide an in vitro release rate of the naltrexone of less than about 90%, or less than about 80%, in about 2 hours. The amount of the sustained-release carrier composition may be effective to provide an in vitro release rate of the naltrexone of less than about 98% in about 4 hours. The amount of the sustained-release carrier composition may be effective to provide an in vitro release rate of the naltrexone of less than about 80% or than about 70% in about 1 hour. In vitro release rate is determined by a standard dissolution test as described above.

A description of representative sustained release carrier materials can be found in the Remington: The Science and Practice of Pharmacy (20th ed, Lippincott Williams & Wilkens Publishers (2003)). Those skilled in the art can formulate sustained-release carrier compositions using routine experimentation informed by the detailed guidance provided herein.

Dosage forms as defined in the claims may be formulated to comprise various excipients, binders, carriers, disintegrants, coatings, etc. Pharmaceutical preparations can be obtained by mixing one or more solid excipients with a pharmaceutical composition as described herein, optionally grinding the resulting mixture, and processing the mixture of granules, after adding suitable auxiliaries, if desired, to obtain pharmaceutical compositions suitable for use in various forms, e.g., as pills, tablets, powders, granules, dragees, capsules, liquids, sprays, gels, syrups, slurries, suspensions and the like, in bulk or unit dosage forms, for oral ingestion by a patient to be treated. Various examples of unit dosage forms are described herein; non-limiting examples include a pill, a tablet, a capsule, a gel cap, and the like. Examples of suitable excipients are listed below, some of which are mentioned above as having particular dissolution properties. Pharmaceutically acceptable carriers or diluents for therapeutic use are well known in the pharmaceutical art, and are described, for example, in Remington: The Science and Practice of Pharmacy (2003). The term "carrier" material or "excipient" herein can mean any substance, not itself a therapeutic agent, used as a carrier, diluent, adjuvant, binder, and/or vehicle for delivery of a therapeutic agent to a subject or added to a pharmaceutical composition to improve its handling or storage properties or to permit or facilitate formation of a dose unit of the composition into a discrete article such as a capsule or tablet suitable for oral administration. Excipients can include, by way of illustration and not limitation, diluents, disintegrants, binding agents, adhesives, wetting agents, polymers, lubricants, glidants, substances added to mask or counteract a disagreeable taste or odor, flavors, dyes, fragrances, and substances added to improve appearance of the composition. The glidants may be one or more of colloidal silicon dioxide, talc, corn starch, DL-leucine, sodium lauryl sulfate, and magnesium, calcium and sodium stearates. The diluents may be one or more of lactose, starch, mannitol, sorbitol, dextrose, microcrystalline cellulose, dibasic calcium phosphate, sucrose-based diluents, confectioner's sugar, monobasic calcium sulfate monohydrate, calcium sulfate dihydrate, calcium lactate trihydrate, dextrates, inositol, hydrolyzed cereal solids, amylose, powdered cellulose, calcium carbonate, glycine, or bentonite. Acceptable excipients include lactose, sucrose, starch powder, maize starch or derivatives thereof, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinyl-pyrrolidone, and/or polyvinyl alcohol, saline, dextrose, mannitol, lactose, lecithin, albumin, sodium glutamate, cysteine hydrochloride, and the like. Examples of suitable excipients for soft gelatin capsules include vegetable oils, waxes, fats, semisolid and liquid polyols. Suitable excipients for the preparation of solutions and syrups include, without limitation, water, polyols, sucrose, invert sugar and glucose. The pharmaceutical compositions can additionally include preservatives, solubilizers, stabilizers, wetting agents, emulsifiers, sweeteners, colorants, flavorings, buffers, coating agents, or antioxidants. Dissolution or suspension of the active ingredient in a vehicle such as water or naturally occurring vegetable oil like sesame, peanut, or cottonseed oil or a synthetic fatty vehicle like ethyl oleate or the like may be desired. Buffers, preservatives, antioxidants and the like can be incorporated according to accepted pharmaceutical practice. The compound can also be made in microencapsulated form. If desired, absorption enhancing preparations (for example, liposomes), can be utilized. Those skilled in the art can formulate sustained-release dosage forms containing one or more of the foregoing ingredients by routine experimentation informed by the detailed guidance provided herein.

### Kits

In some embodiments, the present invention may be presented as a kit. The kit may include one or more unit dosage forms as defined in the claims comprising sustained-release naltrexone. The unit dosage forms may be of an oral formulation. The unit dosage forms may comprise tablets. The kit may include a plurality of unit dosage forms.

The kit may include information. The information may be in a form prescribed by a governmental agency regulating the manufacture, use, or sale of pharmaceuticals, which notice is reflective of approval by the agency of the form of the drug for human or veterinary administration. Such information, for example, may be the labeling approved by the U.S. Food and Drug Administration for prescription drugs, or the approved product insert. Dosage forms comprising a sustained-release naltrexone formulation of the invention formulated in a compatible pharmaceutical carrier may also be prepared, placed in an appropriate container, and labeled for treatment of an indicated condition.

The information may comprise instructions to administer the unit dosage form at a dosage of about 4 mg, about 8 mg, about 12 mg, about 16 mg, about 32 mg or about 48 mg. These instructions may be provided in a variety of ways. The information may comprise instructions about when to administer the unit dosage forms. For example, the information may comprise instructions about when to administer the unit dosage forms relative to the administration of another medication or meal.

The information may be provided on a readable medium. The readable medium may comprise a label. The kit may comprise a therapeutic package suitable for commercial sale. The kit may comprise a container. The container can be in any conventional shape or form as known in the art which is made of a pharmaceutically acceptable material, for example a paper or cardboard box, a glass or plastic bottle or jar, a re-sealable bag (for example, to hold a "refill" of tablets for placement into a different container), or a blister pack with individual dosages for pressing out of the pack according to a therapeutic schedule. The container employed can depend on the exact dosage form involved, for example a conventional cardboard box would not generally be used to hold a liquid suspension. It is feasible that more than one container can be used together in a single package to market a single dosage form. For example, tablets may be contained in a bottle which is in turn contained within a box.

The information can be associated with the container, for example, by being: written on a label (e.g., the prescription label or a separate label) adhesively affixed to a bottle containing a dosage form described herein; included inside a container as a written package insert, such as inside a box which contains unit dose packets; applied directly to the container such as being printed on the wall of a box; or attached as by being tied or taped, for example as an instructional card affixed to the neck of a bottle via a string, cord or other line, lanyard or tether type device. The information may be printed directly on a unit dose pack or blister pack or blister card.

### EXAMPLES

The examples below are non-limiting and are merely representative of various aspects of the invention. Examples not falling within the scope of the claims are for reference only.

### EXAMPLE 1

A series of sustained-release naltrexone (5 mg) formulations were prepared. Each of the sustained-release formulations contained a "common excipient". The formulation for the common excipient is shown in **Table 1.** The formulation shown in **Table 2** contained hydroxypropylmethyl cellulose as a sustained release carrier or retardant excipient. The formulation is shown in **Table 3** contained polyethylene oxide as a retardant excipient.

To make the common excipient, the ingredients listed in **Table 1** were placed into a Key KG-5 hi-shear granulator (Key International, Englishtown, NJ) and granulated with a 10% hydroxypropyl cellulose (HPC) solution. The granulation was dried at room temperature to a final LOD of 2.71% and then screened through a 20-mesh sieve.

To manufacture the naltrexone formulations, the active pharmaceutical ingredient (API) was mixed with a colloidal silicon dioxide as a glidant, the extending release polymer (e.g., either HPMC or PolyOx) and the common excipient using a TURBULA mixer. Magnesium stearate was added as a lubricant to the final formulation.

Tablets with a 5Kp harness were compressed to determine the disintegration properties of the tablet. Under the conditions used, the 5Kp tablet did not disintegrate within 30 minutes in USP water. Accordingly, tablets with a higher hardness were compressed to 9Kp using 1/4" round standard concave tooling on a Carver press.

Tablets compressed at 9Kp were produced, and the properties of the tablets are listed in **Table 4.**

**Table 1: Formulation for the Common excipient**

| **Ingredient** | **Amount (Percent)** |
|---|---|
| Hydroxypropyl cellulose (Klucel HXF) | 3.000 |
| Microcrystalline cellulose, NF (Avicel PH 102) | 77.000 |
| Lactose Fast Flo, (Type 316) NF | 20.000 |
| USP water | NA |
| Total | 100.000 |

**Table 2: Formulations for Sustained Release 5 mg Strength Tablets Containing Hydroxypropylmethylcellulose (HPMC)**

| **Ingredient** | **5% HPMC % per tablet** | **10% HPMC % per tablet** | **15% HPMC % per tablet** | **22% HPMC % per tablet** | **44% HPMC % per tablet** | **66% HPMC % per tablet** |
|---|---|---|---|---|---|---|
| Naltrexone (5mg) | 6.667 | 6.667 | 6.667 | 6.667 | 6.667 | 6.667 |
| Hydroxypropylmethylcellulose (Methocel K15 Premium) | 5.000 | 10.000 | 15.000 | 22.000 | 44.333 | 66.000 |
| Common QBQ01 Placebo Granulation | 86.833 | 81.833 | 76.833 | 69.833 | 47.500 | 25.833 |
| Colloidal Silicon Dioxide, NF (Cab-O-Sil M5P) | 1.000 | 1.000 | 1.000 | 1.000 | 1.000 | 1.000 |
| Magnesium Stearate, NF, Ph.Eur.(Vegetable Source)(Grade, 905-G) | 0.500 | 0.500 | 0.500 | 0.500 | 0.500 | 0.500 |

**Table 3: Formulations for Sustained Release 5 mg Strength Tablets Containing Polyethylene Oxide (PolyOx)**

| **Ingredient** | **5% PolyOx % per tablet** | **10% PolyOx % per tablet** | **20% PolyOx % per tablet** | **30% PolyOx % per tablet** | **59% PolyOx % per tablet** | **75% PolyOx % per tablet** |
|---|---|---|---|---|---|---|
| Naltrexone (5mg) | 6.667 | 6.667 | 6.667 | 6.667 | 6.667 | 6.667 |
| Polyethylene Oxide | 5.000 | 10.000 | 20.000 | 30.000 | 59.333 | 75.000 |
| Common QBQ01 Placebo Granulation | 86.833 | 81.833 | 71.833 | 61.833 | 32.500 | 16.833 |
| Colloidal Silicon Dioxide, NF (Cab-O-Sil M5P) | 1.000 | 1.000 | 1.000 | 1.000 | 1.000 | 1.000 |
| Magnesium Stearate, NF, Ph.Eur.(Vegetable Source)(Grade, 905-G) | 0.500 | 0.500 | 0.500 | 0.500 | 0.500 | 0.500 |

**Table 4: In-Process Tablet Data**

| **Formulation** | **Weight (mg) (N=3)** | **Thickness (mm) (N=3)** | **Hardness (Kp) (N=3)** | **Compression force (psi)** |
|---|---|---|---|---|
| 5% HPMC | 75.0 | 2.47 | 9.2 | 850 |
| 10% HPMC | 75.8 | 2.5 | 8.7 | 800 |
| 15% HPMC | 75.5 | 2.52 | 8.7 | 800 |
| 22% HPMC | 74.2 | 2.45 | 9.2 | 850 |
| 44% HPMC | 75.1 | 2.64 | 8.7 | 750 |
| 66% HPMC | 73.9 | 2.65 | 9.6 | 650 |
| 5% PolyOx | 75.7 | 2.48 | 8.3 | 650 |
| 10% PolyOx | 76.6 | 2.53 | 8.9 | 750 |
| 20% PolyOx | 75.9 | 2.54 | 8.7 | 850 |
| 30% PolyOx | 74.5 | 2.53 | 8.9 | 650 |
| 59% PolyOx | 75.1 | 2.64 | 9.3 | 300 |
| 75% PolyOx | 75.9 | 2.84 | 9.4 | 250 |

The following example describes the dissolution profiles of the sustained release naltrexone formulations described above.

### EXAMPLE 2

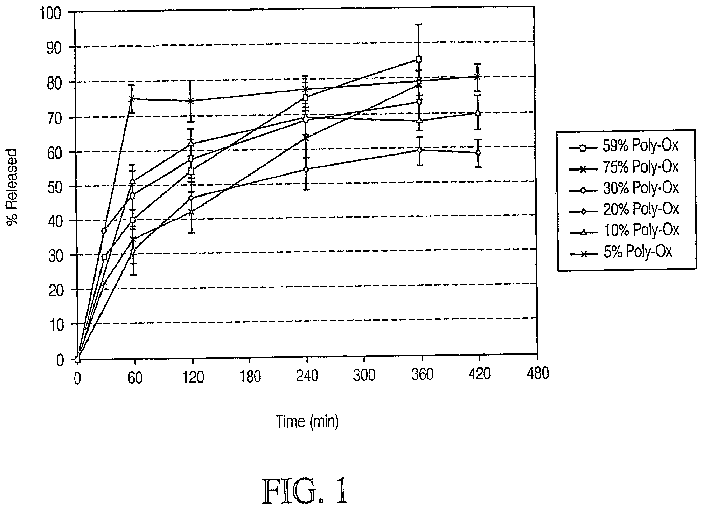

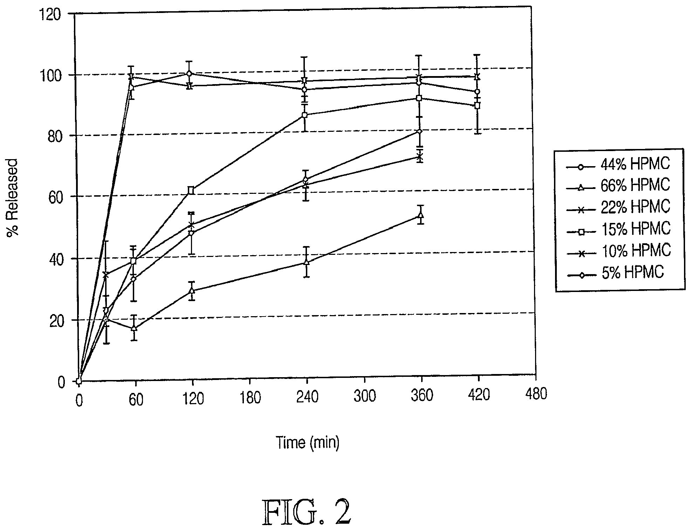

The dissolution measurements for the tablets were completed using a 10-mesh baskets at 100 rpm. Samples were analyzed using a UV-VIS at λₘₐₓ of 280. The dissolution data of the active pharmaceutical ingredient (API) for the HPMC formulations and PolyOx formulations are presented in Tables 5 and 6, respectively. Dissolution data for the HPMC formulations and PolyOx formulations are also plotted in **Figures 1** and **2****,** respectively.

**Table 5: Dissolution Data for HPMC Formulations**

| **Time (min)** | **% API Released 5% HPMC** | **Std. Dev. 5% HPMC** | **% API Released 10% HPMC** | **Std. Dev. 10% HPMC** | **% API Released 15% HPMC** | **Std. Dev. 15% HPMC** | **% API Released 22% HPMC** | **Std. Dev. 22% HPMC** | **% API Released 44% HPMC** | **Std. Dev. 44% HPMC** | **% API Released 66% HPMC** | **Std. Dev. 66% HPMC** |
|---|---|---|---|---|---|---|---|---|---|---|---|---|
| 0 | 0 | 0 | 0 | 0 | 0 | 0 | 0 | 0 | 0 | 0 | 0 | 0 |
| 30 | | | | | | | 35 | 11 | 23 | 5 | 20 | 8 |
| 60 | 96 | 4 | 99 | 4 | 39 | 5 | 39 | 4 | 33 | 7 | 17 | 4 |
| 120 | 100 | 4 | 96 | 1 | 62 | 1 | 51 | 3 | 48 | 7 | 29 | 3 |
| 240 | 94 | 4 | 97 | 8 | 86 | 6 | 63 | 5 | 65 | 3 | 38 | 5 |
| 360 | 96 | 0 | 98 | 7 | 91 | 6 | 72 | 2 | 80 | 5 | 53 | 3 |
| 420 | 93 | 4 | 98 | 7 | 88 | 9 | | | | | | |

**Table 6: Dissolution Data for Polyethylene Oxide Formulations**

| **Time (min)** | **% API Released 5% PolyOx** | **Std. Dev. 5% PolyOx** | **% API Released 10% PolyOx** | **Std. Dev. 10% PolyOx** | **% API Released 20% PolyOx** | **Std. Dev. 20% PolyOx** | **% API Released 30% PolyOx** | **Std. Dev. 30% PolyOx** | **% API Released 59.3% PolyOx** | **Std. Dev. 59.3% PolyOx** | **% API Released 75% PolyOx** | **Std. Dev. 75% PolyOx** |
|---|---|---|---|---|---|---|---|---|---|---|---|---|
| 0 | 0 | 0 | 0 | 0 | 0 | 0 | 0 | 0 | 0 | 0 | 0 | 0 |
| 30 | | | | | | | 37 | 13 | 29 | 1 | 22 | 12 |
| 60 | 75 | 7 | 51 | 5 | 31 | 7 | 47 | 6 | 40 | 3 | 34 | 24 |
| 120 | 74 | 6 | 62 | 4 | 46 | 6 | 57 | 3 | 54 | 1 | 42 | 15 |
| 240 | 77 | 4 | 69 | 5 | 54 | 6 | 68 | 5 | 75 | 4 | 63 | 17 |
| 360 | 79 | 6 | 68 | 3 | 59 | 4 | 73 | 10 | 85 | 10 | 78 | 13 |
| 420 | 80 | 4 | 70 | 5 | 58 | 4 | | | | | | |

### EXAMPLE 3

Sustained-release naltrexone-bupropion tri-layer tablets were made using the ingredients listed in **Table 7** through **Table 9,** in accordance with the general methods for making tri-layer tablets described in U.S. Provisional Patent Application Serial No. 60/865,157, filed November 9, 2006. A sustained-release naltrexone formulation was made by combining the following components:

**Table 7: Naltrexone Blend Formulation**

| **Component** | **mg/tablet** | **mass (g)** |
|---|---|---|
| Naltrexone Hydrochloride | 13.22 | 1983.0 |
| Edetate Disodium, USP | 0.23 | 34.5 |
| Hydroxypropylmethylcellulos e (Methocel K15 Premium CR) | 22.50 | 3375.0 |
| Hydroxypropylcellulose | 11.00 | 1650.0 |
| Lactose Monohydrate NF, Fast Flo 316 | 45.50 | 6825.0 |
| Microcrystalline cellulose (Avicel PH102) | 128.95 | 19342.5 |
| Colloidal silicon dioxide, NF | 2.30 | 345.0 |
| Magnesium Stearate, NF | 1.30 | 195.0 |
| **Total** | **225.0** | **33750.0** |

Thus, the sustained-release naltrexone formulation includes 10% HPMC. A bupropion blend was made by combining the following components:

**Table 8: Bupropion Blend Formulation**

| **Component** | **mg/tablet** | **mass (g)** |
|---|---|---|
| Bupropion Hydrochloride Granulation | 315.00 | 47250.0 |
| Magnesium Stearate | 2.00 | 300.0 |
| **Total** | **317.00** | **47550.0** |

An inert layer blend was made by combining the following components:

**Table 9: Inert Blend Formulation**

| **Component** | **mg/tablet** | **mass (g)** |
|---|---|---|
| Anhydrous Lactose | 30.00 | 4500.0 |
| Microcrystalline cellulose (Avicel PH101) | 84.70 | 12705.0 |
| Crospovidone | 3.60 | 540.0 |
| Magnesium Stearate | 1.20 | 180.0 |
| FDC Blue #2 Aluminum Lake | 0.50 | 75.0 |
| **Total** | **120.0** | **18000.0** |

The sustained-release naltrexone formulation, bupropion blend and inert layer blends were used to form 150 tri-layer tablets with the naltrexone and bupropion layers on opposite sides of the inert layer, such that each tablet was 662.00 mg. The tablets each contained 11.94 mg of naltrexone (13.22 mg naltrexone hydrochloride).

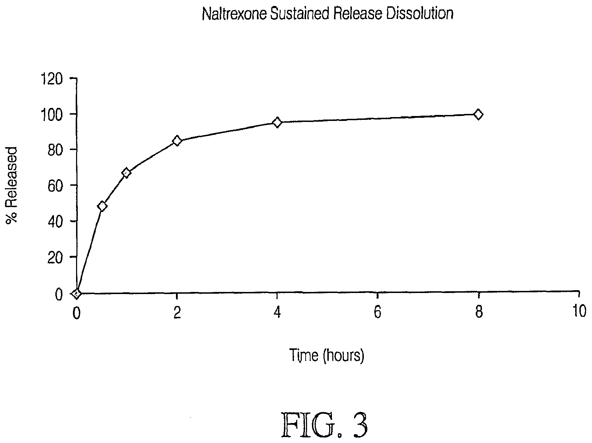

The dissolution data of naltrexone for the tablets is presented in **Table 10.** Dissolution data is also plotted in **Figure 3****.**

**Table 10: Dissolution Data for Naltrexone-Bupropion Tablets**

| **Time (Hours)** | **Naltrexone Released (Wt %)** |
|---|---|
| 0 | 0 |
| 0.5 | 48 |
| 1 | 67 |
| 2 | 85 |
| 4 | 95 |
| 8 | 99 |

### EXAMPLE 4

A single center, double-blind, crossover study of immediate release naltrexone and sustained release naltrexone was conducted for 40 healthy obese volunteers.

Subjects were randomized to receive a naltrexone sustained-release formulation or an immediate-release formulation in a 1:1 ratio. Subjects were administered 40 mg of SR Naltrexone and 36 mg of IR Naltrexone. Subjects were randomly assigned to receive the sustained-release and immediate-release in one of two sequences: half of the subjects received the sustained-release formulation in period 1 followed by the immediate-release formulation in period 2; the remaining subjects received the treatments in the reverse order.

The administration of study drug in each period was separated by a five-day washout period. Serial blood samples were collected at predose and at 0.167, 0.33, 0.5, 0.67, 0.83, 1, 1.25, 1.5, 2, 3, 4, 6, 8, 10, 12, 16, 24, 36, 48, 60 and 72 hours post-dose. Samples were analyzed for the concentrations of naltrexone and 6-beta naltrexol by validated LCMS/MS methods. In addition to spontaneously collected adverse events, information on side effects was collected using the subject rated UKU Side Effect Rating Scale. The scale was administered predose, at 8, 24 and 72 hours post-dose.

The concentration-time data were imported directly into WinNonlin (Version 5.0, Pharsight Corporation). Data were analyzed by noncompartmental methods using WinNonlin using Model 200 for extravascular input.

Concentration-time data that were BLLOQ were excluded from calculations prior to data summarization and pharmacokinetic analysis. Data were summarized by scheduled (nominal) sampling times. Actual sampling times were used if the deviation from nominal was considered significant.

The following pharmacokinetic parameters were estimated for naltrexone and 6-beta naltrexol concentrations for each subject and each treatment:
Cₘₐₓ = measured maximal concentration
Tₘₐₓ = time to reach maximum concentration
Ke = terminal rate constant, estimated by log-linear regression
AUC(0-t) = area under the concentration-time curve calculated by the linear trapezoidal rule from time 0 to the time of last sample with a quantifiable concentration (Cₜ)
AUC(0-∞) = area under the concentration time curve from time 0 extrapolated to infinity, calculated as AUC (0-t) + Cₜ/Kₑ
T_{½} = terminal half-life, calculated as Ln(2)/Ke

The AUC is used to describe the extent of drug absorption. The rate of absorption is characterized by Cₘₐₓ and Tₘₐₓ. Ke and T_{½} describe the kinetics in the terminal phase, which, for many substances, is governed by elimination processes. Pharmacokinetic parameters were estimated for all cases.

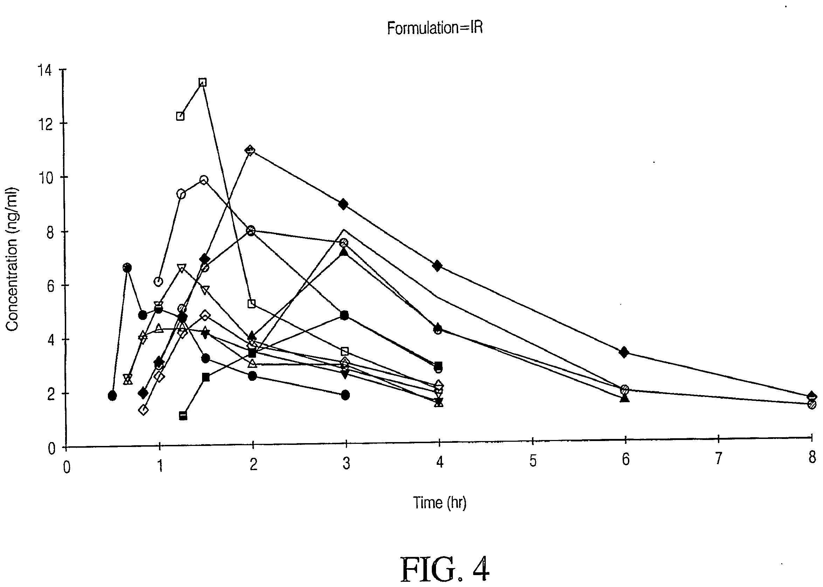

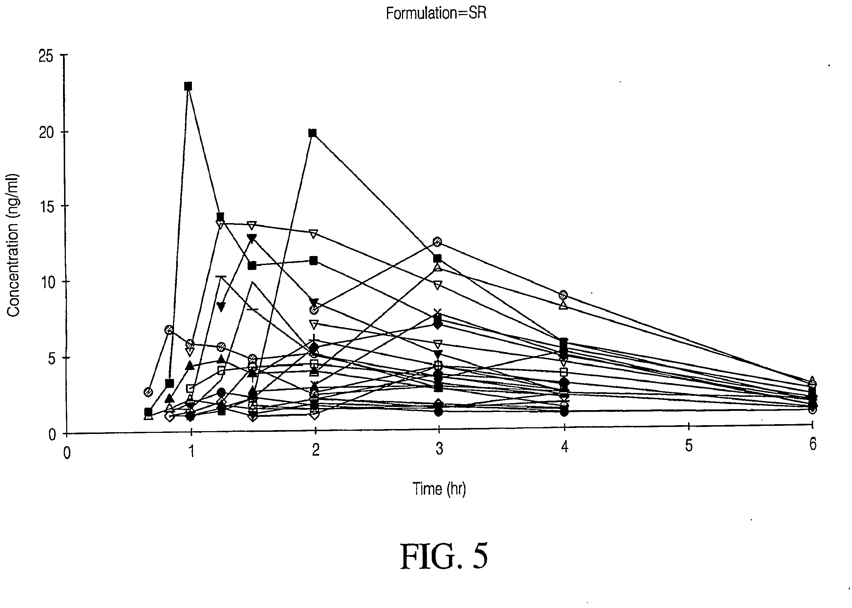

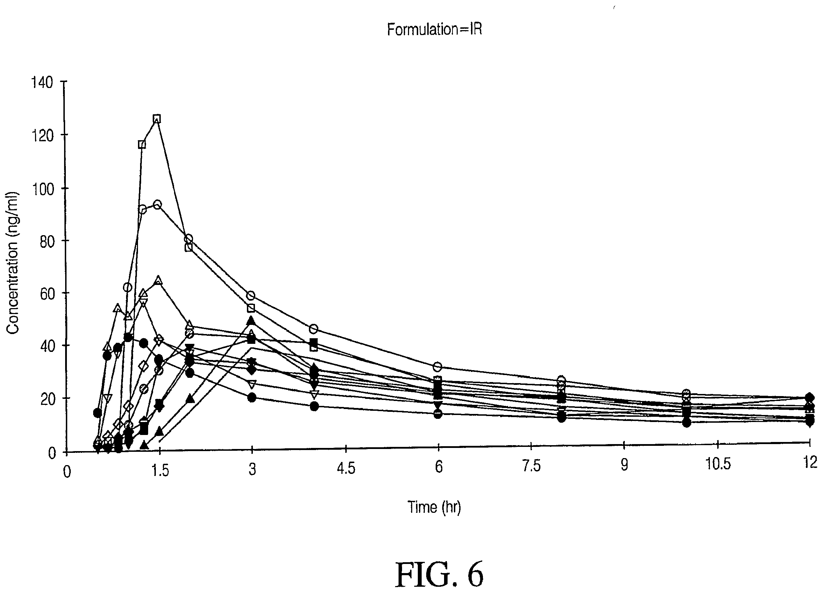

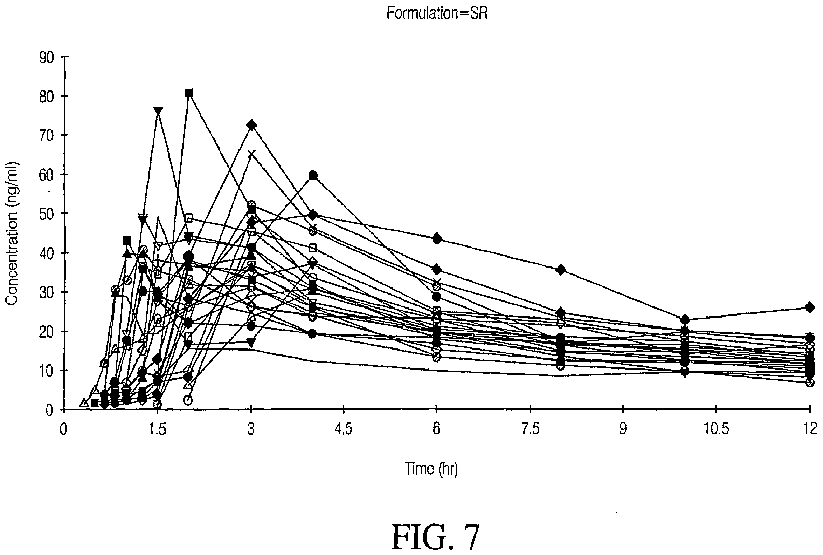

**Figures 4** and **5** show individual plasma concentration time curves of naltrexone in subjects receiving Naltrexone IR and in subjects receiving Naltrexone SR, respectively. **Figures 6** and **7** show individual plasma concentration time curves of 6-beta naltrexol in subjects receiving Naltrexone IR and in subjects receiving Naltrexone SR, respectively.

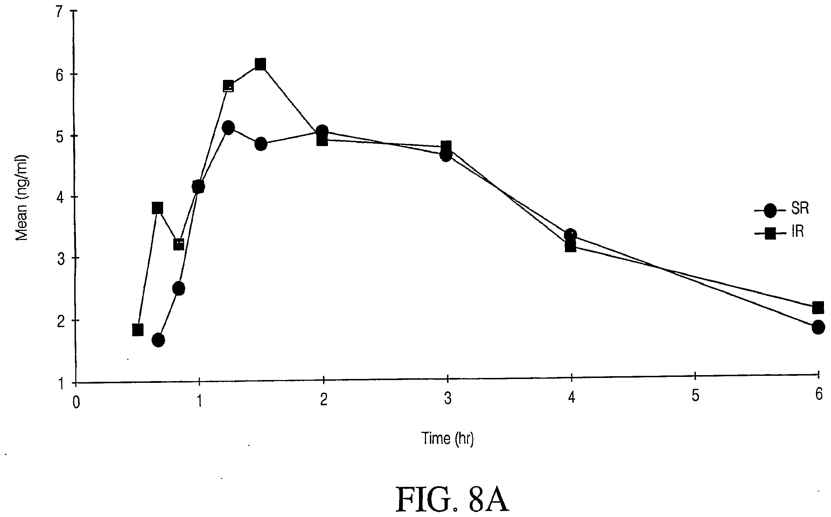

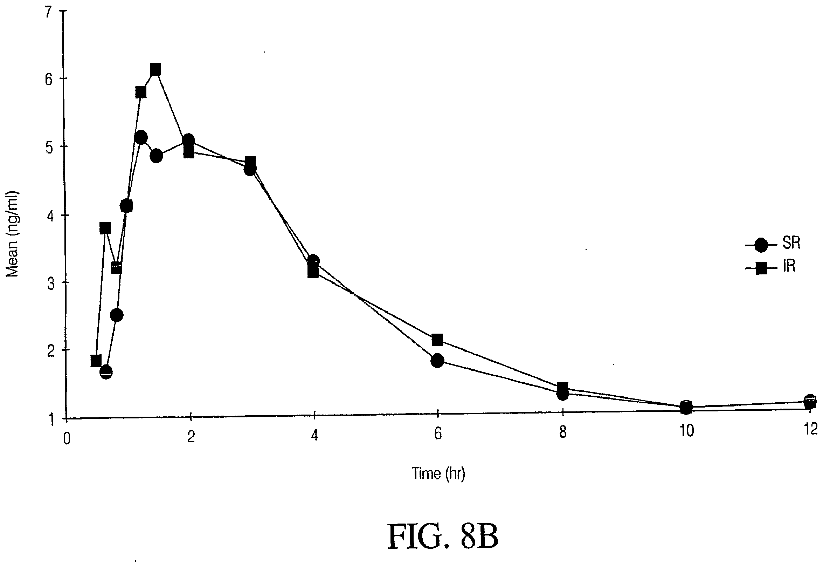

**Figure 8A** shows the average plasma concentration time curves of naltrexone in subjects receiving Naltrexone SR (circle) or Naltrexone IR (square). **Figure 8B** shows the entire plasma concentration time curve. The Naltrexone Cₘₐₓ is higher for patients receiving Naltrexone IR than for those receiving Naltrexone SR though the area under the curve is comparable between both conditions. These results are dose normalized and quantified in **Table 11.**

**Table 11. Summary of Pharmacokinetic Parameters in Subjects Receiving Naltrexone SR or Naltrexone IR**

| **Analyte** | **Formulation** | **Cₘₐₓ (ng/mL)** | **Tₘₐₓ (hr)** | **AUCₗₐₛₜ (ng^{∗}hr/mL)** | **T_{1/2} (hr)** |
|---|---|---|---|---|---|
| Naltrexone | SR | 5.10 | 1.25 | 27.48 | 4.01 |
| | *Dose Normalized* | *4.59* | | *24.7* | |
| | IR | 6.11 | 1.50 | 24.91 | 3.27 |
| 6-beta naltrexol | SR | 37.45 | 3.00 | 486.00 | 16.55 |
| | *Dose Normalized* | *33.7* | | *437.4* | |
| | IR | 43.39 | 1.5 | 496.95 | 16.99 |

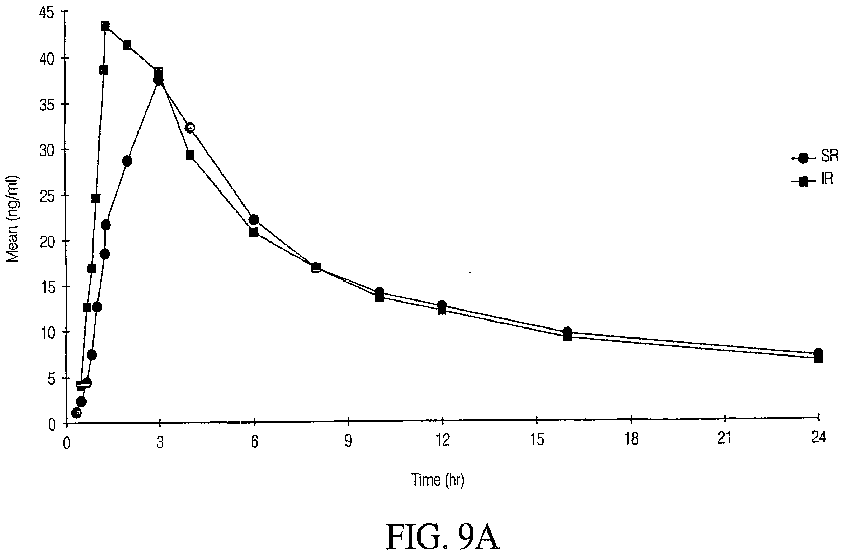

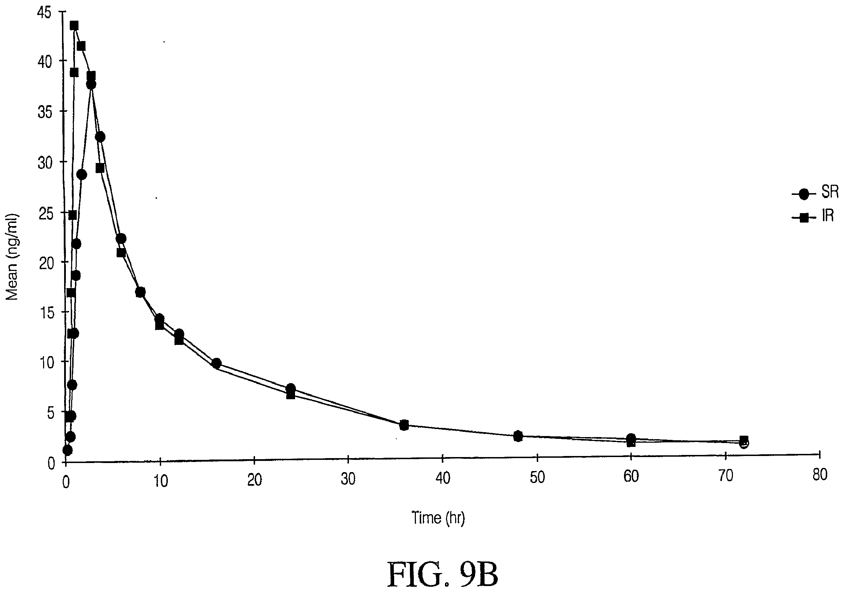

**Figure 9A** shows the average plasma concentration time curves of 6-beta naltrexol in subjects receiving Naltrexone SR (circle) or Naltrexone IR (square). **Figure 9B** shows the entire plasma concentration time curve. The 6-beta naltrexol Cₘₐₓ is higher for patients receiving Naltrexone IR than for those receiving Naltrexone SR though the area under the curve is comparable between both conditions. These results are dose normalized and quantified in **Table 11.**

**Table 12** provides the adverse events reported by patients receiving Naltrexone SR and by patients receiving Naltrexone IR. The adverse events are analyzed across patients for each of the treatment conditions. These results are summarized in **Table 13.** Individuals receiving Naltrexone SR were less likely to report an adverse event (45% versus 48%) and were also less likely to report more than one adverse event (13% versus 23%) than individuals receiving Naltrexone IR.

**Table 12. Summary of Individual Adverse Events.**

| **Subject** | **Period** | **Treatment** | **AE YM** | **Adverse Event(s)** | | | |
|---|---|---|---|---|---|---|---|
| 1 | 1 | SR | Yes | Nausea | | | |
| 4 | 1 | SR | Yes | Headache | Anemia | | |
| 5 | 1 | SR | Yes | Elevated platelet count | | | |
| 11 | 1 | SR | Yes | Hyperglycemia | | | |
| 16 | 1 | SR | Yes | Cramps, menstrual | | | |
| 17 | 2 | SR | Yes | Pyuria | | | |
| 19 | 1 | SR | Yes | Nausea | Elevated Leukocyte Esterase | Pyuria | |
| 22 | 1 | SR | Yes | Stomach pain | | | |
| 27 | 2 | SR | Yes | Hypophosphatemia | | | |
| 32 | 1 | SR | Yes | Hypertension | | | |
| 34 | 1 | SR | Yes | Headache | Headache | | |
| 35 | 1 | SR | Yes | Hyperleukemia | | | |
| 36 | 1 | SR | Yes | Nausea | Stomach Cramps | | |
| 37 | 1 | SR | Yes | Tingle in lips | | | |
| 39 | 1 | SR | Yes | Nausea | Diarrhea | Dizziness | |
| 40 | 2 | SR | Yes | Hematuria | Hypophosphatemia | | |
| 29 | 2 | SR | Yes | Increased TSH | | | |
| 1 | 2 | IR | Yes | Pyuria | | | |
| 2 | 2 | IR | Yes | Intermittent Diarrhea | Pyuria | Hematuria | |
| 4 | 2 | IR | Yes | Anemia | | | |
| 5 | 2 | IR | Yes | Elevated platelet count | | | |
| 7 | 2 | IR | Yes | Elevated blood pressure | | | |
| 11 | 2 | IR | Yes | Nausea | Hypertension | Proteinuria | Hematuria Hyperplycemia |
| 13 | 2 | IR | Yes | Hyperglycemia | | | |
| 15 | 2 | IR | Yes | Hyperleukemia | | | |
| 16 | 2 | IR | Yes | Blood in urine | | | |
| 18 | 2 | IR | Yes | Elevated Leukocyte Esterase | Pyuria | Leucocytosis | |
| 19 | 2 | IR | Yes | Nausea | | | |
| 22 | 2 | IR | Yes | Stomach pain | Vomited | Elevated Leukocyte Esterase | |
| 23 | 1 | IR | Yes | Hyperglycemia | | | |
| 25 | 2 | IR | Yes | Triglyceride | | | |
| 27 | 1 | IR | Yes | Hypophosphatemia | | | |
| 31 | 2 | IR | Yes | Hypertension | | | |
| 32 | 2 | IR | Yes | Hypertension | Increased TSH | | |
| 34 | 2 | IR | Yes | Elevated ALT | Increased TSH | | |
| 35 | 2 | IR | Yes | Hyperglycemia | | | |
| 36 | 2 | IR | Yes | Numbness in lips | Hyperglcemia | | |

**Table 13. Summary of Adverse Events, Gastrointestinal and Headache, in Subjects Receiving Naltrexone SR and Naltrexone IR.**

| **Item** | **Formulation** | **%** | | |
|---|---|---|---|---|
| % Subjects Reporting AE | SR | 45% | 18 | 40 |
| | IR | 48% | 19 | 40 |
| % Subjects Reporting >1 AE | SR | 13% | 5 | 40 |
| | IR | 23% | 9 | 40 |
| % Subjects Reporting AEs in Both Periods | | 33% | 13 | 39 |
| | | | | |
| % Subjects GI Reporting AE | SR | 13% | 5 | 40 |
| | IR | 10% | 4 | 40 |
| % Subjects Reporting >1 GI AE | SR | 5% | 2 | 40 |
| | IR | 3% | 1 | 40 |
| % Subjects Reporting GI AEs in Both Periods | (19,22,36) | 8% | 3 | 40 |
| | | | | |
| % Subjects GI Reporting Headache AE | SR | 5% | 2 | 40 |
| | IR | 0% | 0 | 40 |
| | | | | |
| % Subjects Reporting Headache AEs in Both Periods | | 0% | 0 | 40 |

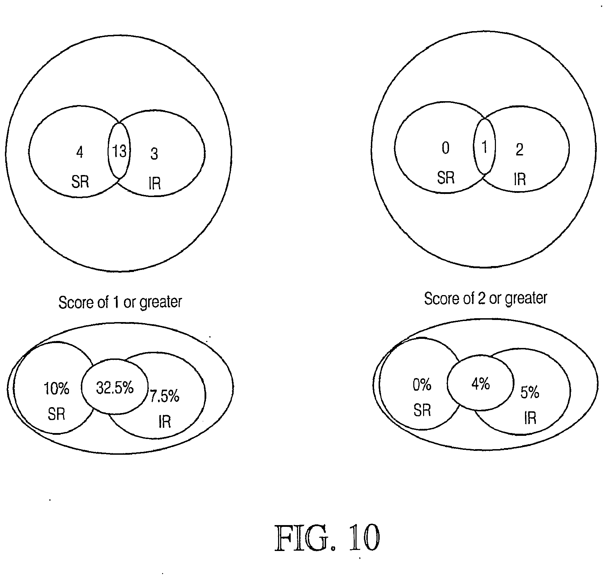

**Figure 10** is a schematic illustrating the population of subjects reporting nausea and vomiting using the UKU Adverse Event Rating Scale. Individuals receiving Naltrexone SR were less likely to report severe nausea (score of 2 or greater) than those receiving Naltrexone IR.

Those skilled in the art recognize that the incidence of adverse events reported using the UKU Adverse Event Rating Scale is typically higher than self-reporting. Patients have been reported to be more likely to report adverse events when prompted to report adverse events (Sheftell FD, Feleppa M, Tepper SJ, Rapoport AM, Ciannella L, Bigal ME. Assessment of adverse events associated with triptans-methods of assessment influence the results. Headache: The Journal of Head and Face Pain 44 (10), 978-982). Thus, the incidence of adverse events obtained by the methods used in this example is not directly comparable to the incidence of adverse events obtained by the methods described in Example 5.

### EXAMPLE 5

A randomized double-blind, parallel, multiple dose study compared the pharmacokinetics of a naltrexone SR / bupropion SR combination product with a naltrexone IR / bupropion SR combination product.

Naltrexone SR/ bupropion SR group: Each capsule contained two and one-half 5 mg mini tablets (prepared in accordance with Example 1) of sustained release naltrexone plus one 90 mg bupropion HCl SR tablet (total dose of naltrexone SR 12.5 mg/bupropion SR 90 mg per capsule) which was titrated over 7 days to. a total daily dose of naltrexone SR 37.5 mg / bupropion SR 270 mg.

Naltrexone IR/bupropion SR group: Each capsule contained capsules containing one 12 mg naltrexone HCl IR tablet plus one 90 mg bupropion HCl SR tablet (total dose of 12 mg naltrexone IR/ 90 mg bupropion SR per capsule) which was titrated over 7 days to a final daily dose of naltrexone IR 36 mg / bupropion SR 270mg. The naltrexone IR formulation is formulated to have a pharmacokinetic profile that is substantially similar to the REVIA® brand of naltrexone hydrochloride.

Healthy obese volunteers randomly received one of the two drug administrations. A total of 60 subjects were expected to enroll, 59 subjects completed the study.

Blood was drawn on Day 1 (0.5, 1, 2, 4, 6, 8, 10, and 12hrs) with trough samples drawn on Days 8 and 15. Plasma samples were drawn from each subject to determine naltrexone, 6-beta-naltrexol, bupropion, hydroxybupropion, threohydroxybupropion, and erthyrohydroxybupropion concentrations. Plasma samples were analyzed using the SFBCi / Anapharm validated methods for naltrexone and 6-beta naltrexol. The concentration-time data were imported directly into WinNonlin (Version 5.0, Pharsight Corporation). Data were analyzed by noncompartmental methods using WinNonlin using Model 200 for extravascular input.

Concentration-time data that were BLLOQ were excluded from calculations prior to data summarization and pharmacokinetic analysis. Data were summarized by scheduled (nominal) sampling times. Actual sampling times were used if the deviation from nominal was considered significant.

Pharmacokinetic parameters measured herein are known to those skilled in the art. Blood plasmas were collected at specific intervals relative to the administration of a combination product. The plasma was analyzed to determine the concentration of a compound (e.g., naltrexone). Cₘₐₓ indicates the maximum blood plasma concentration of the compound after administration. Tₘₐₓ indicates the time at which the maximum blood plasma concentration of the compound (Cₘₐₓ) was reached. AUC indicates the area under the curve of the concentration as a function of time following administration. AUCₗₐₛₜ indicates the area under the plasma-concentration curve from the time of administration until the time of the last measurable concentration.

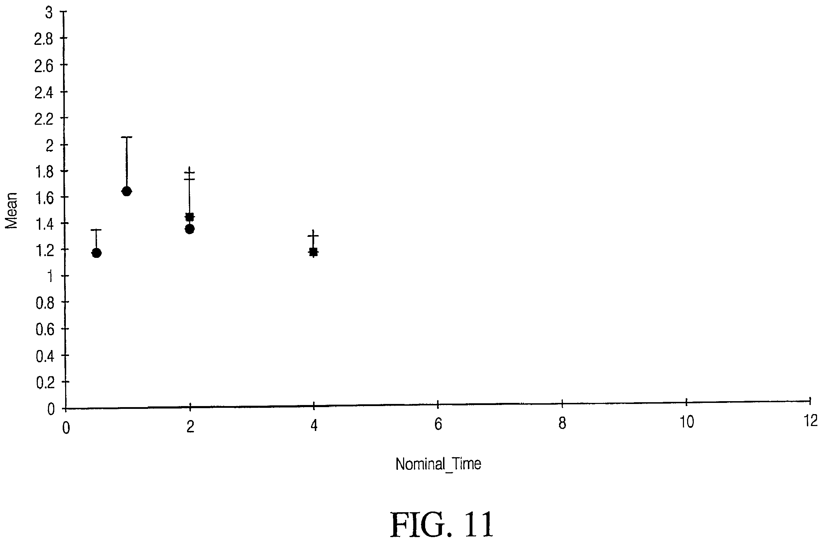

**Table 14** and **Figure 11** compare average naltrexone plasma concentrations in subjects receiving treatments of naltrexone IR and bupropion SR (IR/SR; circles) to that in subjects receiving treatments of naltrexone SR and bupropion SR (SR/SR; squares). The single dose of naltrexone at the onset of the titration schedule of 12 (IR) or 12.5 (SR) produces plasma concentrations during the first 24 hours that are primarily below the lower limit of quantitation (BLLOQ).

**Table 14. Mean plasma concentrations of naltrexone in subjects receiving naltrexone IR in combination with bupropion SR (IR/SR) or naltrexone SR in combination with bupropion SR (SR/SR).**

| **Treatment** | **Time (hr)** | **N** | **Mean (ng/mL)** | **SD (ng/mL)** | **CV%** |
|---|---|---|---|---|---|
| **IR/SR** | 0 | 0 | NC | NC | NC |
| | 0.5 | 8 | 1.17 | 0.18 | 15.45 |
| | 1 | 18 | 1.64 | 0.41 | 25.28 |
| | 2 | 10 | 1.36 | 0.37 | 27.44 |
| | 4 | 0 | NC | NC | NC |
| | 6 | 0 | NC | NC | NC |
| | 8 | 0 | NC | NC | NC |
| | 10 | 0 | NC | NC | NC |
| | 12 | 0 | NC | NC | NC |
| | 168 | 0 | NC | NC | NC |
| | 336 | 1 | 6.78 | NC | NC |
| **SR/SR** | 0 | 0 | NC | NC | NC |
| | 0.5 | 0 | NC | NC | NC |
| | 1 | 0 | NC | NC | NC |
| | 2 | 11 | 1.44 | 0.34 | 23.59 |
| | 4 | 3 | 1.17 | 0.12 | 10.45 |
| | 6 | 0 | NC | NC | NC |
| | 8 | 0 | NC | NC | NC |
| | 10 | 0 | NC | NC | NC |
| | 12 | 0 | NC | NC | NC |
| | 168 | 1 | 1.18 | NC | NC |
| | 336 | 2 | 2.55 | 1.64 | 64.1 |

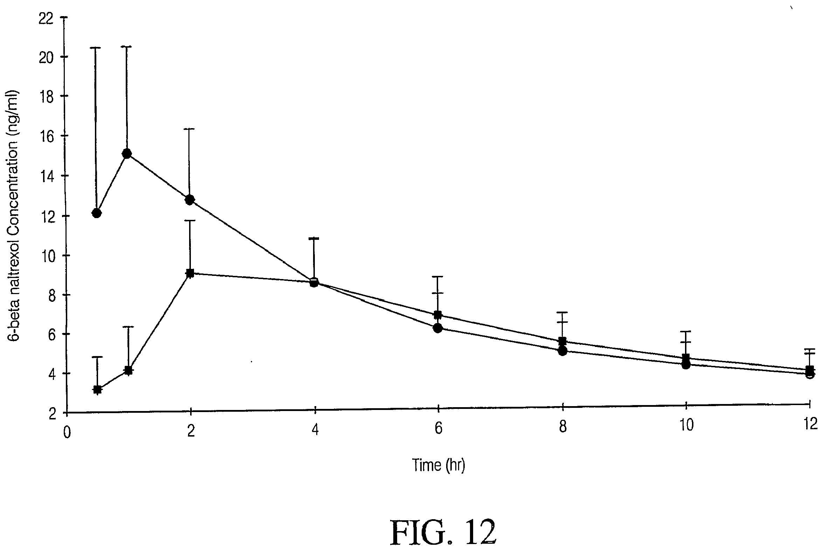

**Table 15** and **Figure 12** compare average 6-beta naltrexol plasma concentrations in subjects receiving treatments of naltrexone IR and bupropion SR (squares) to that in subjects receiving treatments of naltrexone SR and bupropion SR (circles). The plasma concentrations associated with the SR/SR treatment are lower during the first few hours following administration than those associated with the IR/SR treatment. As reported in **Table 16,** the SR/SR treatment is associated with a lower Cₘₐₓ but a similar AUC as compared to the IR/SR treatment.

**Table 15. Mean plasma concentrations of 6-beta naltrexol in subjects receiving naltrexone IR in combination with bupropion SR (IR/SR) or naltrexone SR in combination with bupropion SR (SR/SR).**

| **Treatment** | **Time (hr)** | **N** | **Mean (ng/ml)** | **SD (ng/ml)** | **CV%** |
|---|---|---|---|---|---|
| **IR/SR** | 0 | 0 | NC | NC | NC |
| | 0.5 | 27 | 12.14 | 8.3 | 68.33 |
| | 1 | 30 | 15.05 | 5.44 | 36.15 |
| | 2 | 30 | 12.74 | 3.55 | 27.87 |
| | 4 | 30 | 8.45 | 2.3 | 27.21 |
| | 6 | 30 | 6.11 | 1.8 | 29.43 |
| | 8 | 30 | 4.86 | 1.45 | 29.81 |
| | 10 | 30 | 4.09 | 1.17 | 28.59 |
| | 12 | 30 | 3.56 | 1.05 | 29.54 |
| | 168 | 27 | 10.59 | 4.13 | 39.03 |
| | 336 | 27 | 15.66 | 6.71 | 42.88 |
| **SR/SR** | 0 | 0 | NC | NC | NC |
| | 1 | 13 | 3.2 | 1.65 | 51.66 |
| | 1 | 27 | 4.16 | 2.21 | 52.99 |
| | 2 | 29 | 9.05 | 2.69 | 29.74 |
| | 4 | 29 | 8.51 | 2.17 | 25.47 |
| | 6 | 29 | 6.77 | 1.95 | 28.84 |
| | 8 | 29 | 5.37 | 1.49 | 27.82 |
| | 10 | 29 | 4.43 | 1.35 | 30.53 |
| | 12 | 29 | 3.72 | 1.09 | 29.37 |
| | 168 | 28 | 11.21 | 4.54 | 40.52 |
| | 336 | 26 | 18.67 | 10.74 | 57.52 |

**Table 16. Mean pharmacokinetic parameters for 6-beta naltrexol on Day 1 in subjects receiving naltrexone IR in combination with bupropion SR (IR/SR) or naltrexone SR in combination with bupropion SR (SR/SR).**

| **Parameter** | **IR/SR** | | | | **SR/SR** | | | |
|---|---|---|---|---|---|---|---|---|
| | **N** | **Mean** | **SD** | **CV%** | **N** | **Mean** | **SD** | **CV%** |
| Tmax | 30 | 1.117 | 0.536 | 48 | 29 | 2.621 | 0.942 | 35.9 |
| Cmax | 30 | 16.731 | 6.391 | 38.2 | 29 | 9.686 | 2.323 | 24 |
| AUClast | 30 | 86.658 | 23.457 | 27.1 | 29 | 71.623 | 16.711 | 23.3 |
| AUCINF_ obs | 30 | 118.445 | 35.191 | 29.7 | 29 | 115.771 | 45.398 | 39.2 |
| HL_Lambda_z | 30 | 5.989 | 1.796 | 30 | 29 | 7.838 | 4.58 | 58.4 |

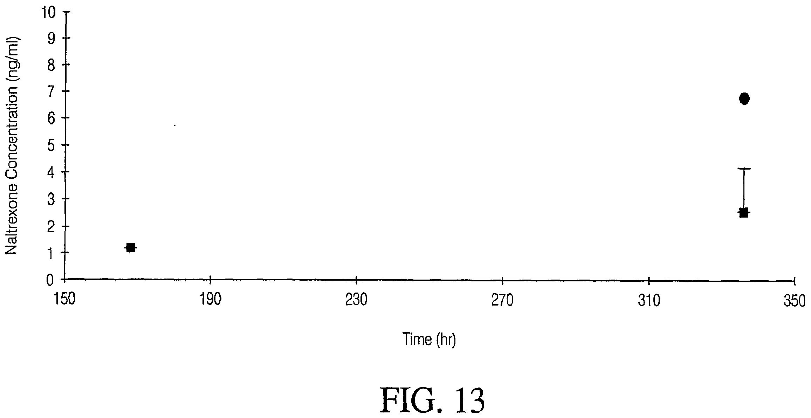

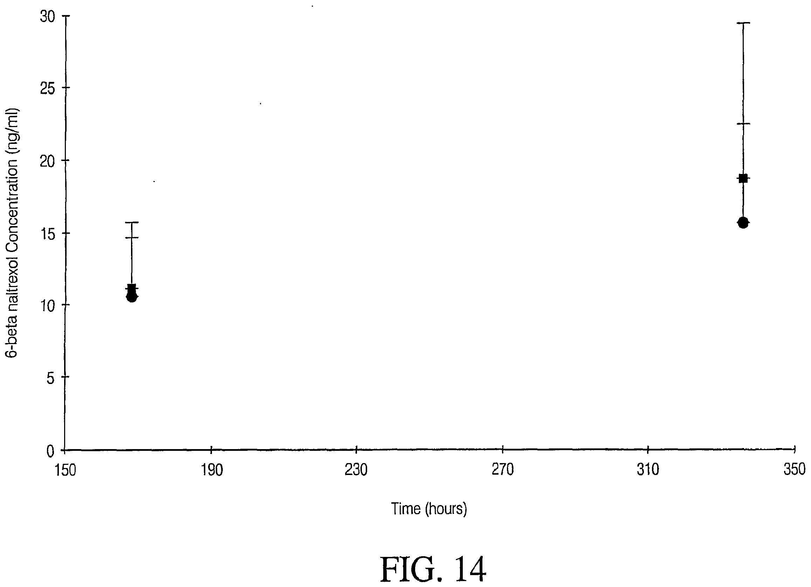

**Figures 13** and **14** compare the trough plasma concentrations of naltrexone and 6-beta naltrexol, respectively, in subjects receiving treatments of naltrexone IR and bupropion SR (circles) to that in subjects receiving treatments of naltrexone SR and bupropion SR (squares). For naltrexone, the trough levels on Day 8 and Day 15 are primarily BLLOQ, with the exception of 1 subject in the IR group, and 2 subjects in the SR group. For 6-beta naltrexol, the trough levels of Day 8 and Day 15 are similar between treatment groups.

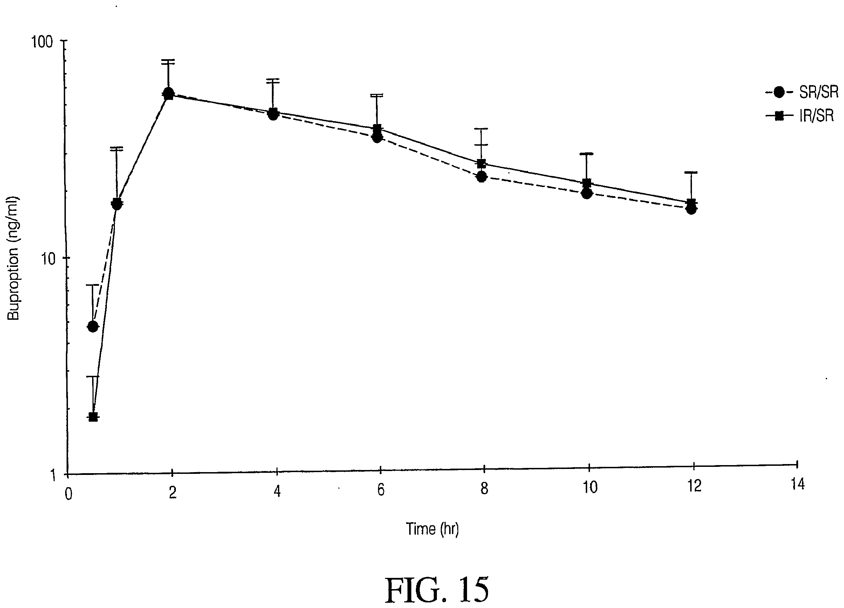

**Table 17** and **Figure 15** compare average bupropion plasma concentrations in subjects receiving treatments of naltrexone IR and bupropion SR (squares) to that in subjects receiving treatments of naltrexone SR and bupropion SR (circles). The plasma concentration profiles are similar across the treatment conditions.

**Table 17. Mean Buproprion Plasma Concentrations in subjects receiving bupropion SR in combination with naltrexone IR (IR/SR) or in combination with naltrexone SR (SR/SR).**

| **Treatment** | **Time (hr)** | **N** | **Mean (ng/ml)** | **SD (ng/ml)** | **CV%** |
|---|---|---|---|---|---|
| **IR/SR** | 0 | 0 | NC | NC | NC |
| | 0.5 | 6 | 1.83 | 0.99 | 54.07 |
| | 1 | 23 | 17.62 | 13.86 | 78.66 |
| | 2 | 23 | 54.23 | 21.64 | 39.9 |
| | 4 | 23 | 44.83 | 16.83 | 37.53 |
| | 6 | 23 | 37.1 | 16.32 | 43.99 |
| | 8 | 23 | 25.29 | 11.28 | 44.59 |
| | 10 | 23 | 20.1 | 7.89 | 39.27 |
| | 12 | 23 | 16.03 | 6.44 | 40.19 |
| **SR/SR** | 0 | 0 | NC | NC | NC |
| | 0.5 | 7 | 4.77 | 2.62 | 54.95 |
| | 1 | 20 | 17.14 | 13.41 | 78.23 |
| | 2 | 20 | 55.04 | 24.96 | 45.35 |
| | 4 | 20 | 43.96 | 20.3 | 46.18 |
| | 6 | 20 | 33.77 | 18.18 | 53.83 |
| | 8 | 20 | 21.98 | 8.77 | 39.89 |
| | 10 | 20 | 18.13 | 9.43 | 52.02 |
| | 12 | 20 | 15.22 | 7.03 | 46.18 |

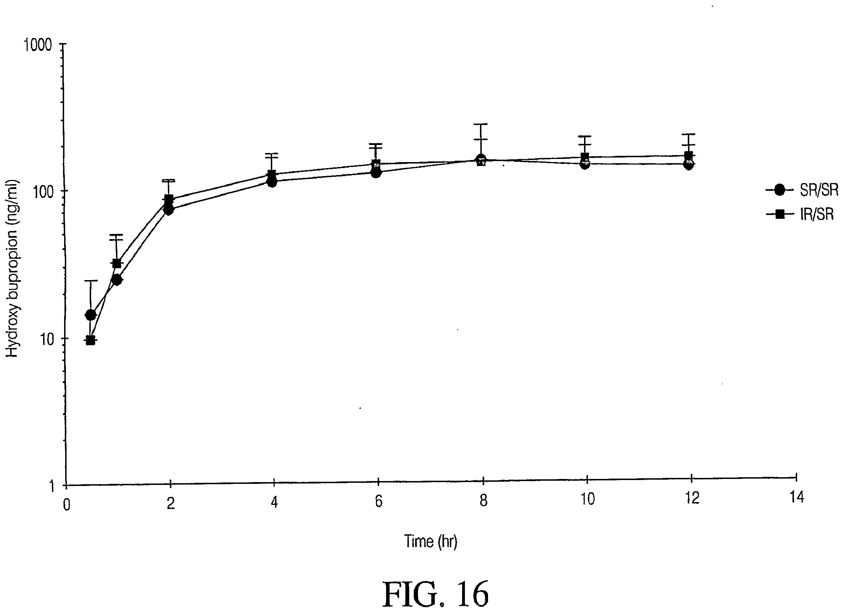

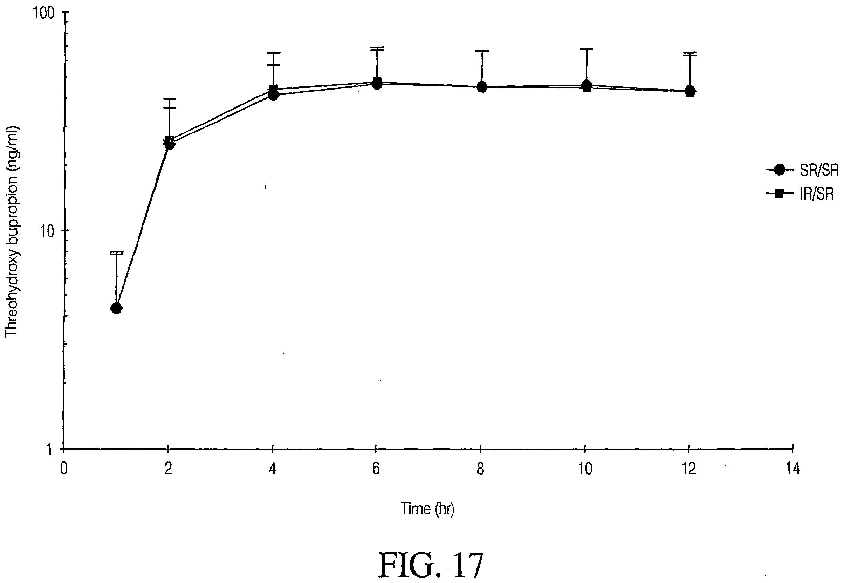

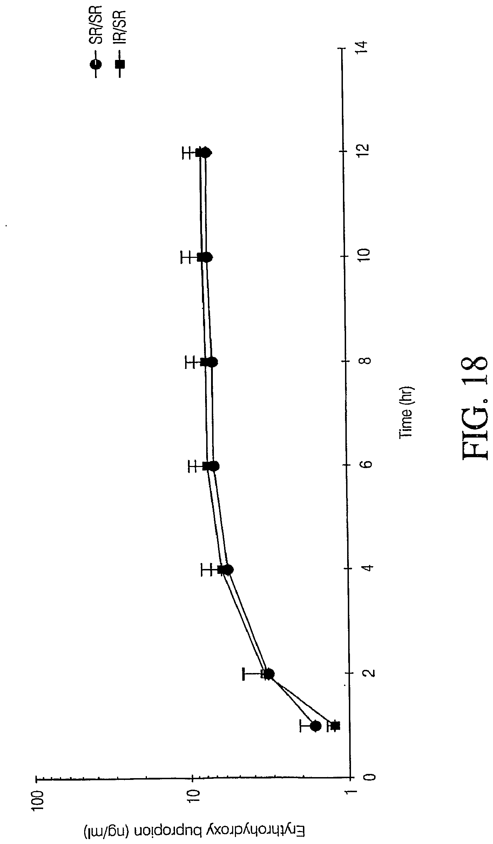

**Tables 18-20** and **Figures 16-18** compare average plasma concentrations for the bupropion metabolites of hydroxybupropion, threohydroxybupropion and erythrohydroxybupropion in subjects receiving treatments of naltrexone IR and bupropion SR (squares) to that in subjects receiving treatments of naltrexone SR and bupropion SR (circles). The plasma concentration profiles are similar across the treatment conditions.

**Table 18. Mean plasma concentrations of the bupropion metabolite hydroxybupropion in subjects receiving bupropion SR in combination with naltrexone IR (IR/SR) or in combination with naltrexone SR (SR/SR).**

| **Treatment** | **Time (hr)** | **N** | **Mean (ng/ml)** | **SD (ng/ml)** | **CV%** |
|---|---|---|---|---|---|
| **IR/SR** | 0 | 0 | NC | NC | NC |
| | 0.5 | 7 | 9.72 | 4.43 | 45.59 |
| | 1 | 26 | 32.41 | 17.36 | 53.58 |
| | 2 | 26 | 85.82 | 31.03 | 36.16 |
| | 4 | 27 | 125.34 | 47.16 | 37.63 |
| | 6 | 27 | 142.53 | 56.45 | 39.6 |
| | 8 | 27 | 147.57 | 59.78 | 40.51 |
| | 10 | 27 | 155.42 | 61.9 | 39.82 |
| | 12 | 27 | 157.17 | 62.65 | 39.86 |
| **SR/SR** | 0 | 0 | NC | NC | NC |
| | 0.5 | 6 | 14.48 | 10.64 | 73.45 |
| | 1 | 24 | 25.07 | 21.07 | 84.02 |
| | 2 | 24 | 74.36 | 39.09 | 52.56 |
| | 4 | 25 | 112.29 | 50.81 | 45.24 |
| | 6 | 25 | 127.44 | 58.1 | 45.59 |
| | 8 | 25 | 150.85 | 114.97 | 76.22 |
| | 10 | 25 | 139.93 | 50.44 | 36.05 |
| | 12 | 25 | 137.81 | 49.48 | 35.9 |

**Table 19. Mean plasma concentrations of the bupropion metabolite threohydroxybupropion in subjects receiving bupropion SR in combination with naltrexone IR (IR/SR) or in combination with naltrexone SR (SR/SR).**

| **Treatment** | **Time (hr)** | **N** | **Mean (ng/ml)** | **SD (ng/ml)** | **CV%** |
|---|---|---|---|---|---|
| **IR/SR** | 0 | 0 | NC | NC | NC |
| | 0.5 | 0 | NC | NC | NC |
| | 1 | 20 | 4.44 | 3.41 | 76.95 |
| | 2 | 27 | 26.16 | 13.99 | 53.48 |
| | 4 | 27 | 44.57 | 21.2 | 47.57 |
| | 6 | 27 | 48.28 | 21.57 | 44.68 |
| | 8 | 27 | 45.88 | 21.29 | 46.4 |
| | 10 | 27 | 45.39 | 22.2 | 48.92 |
| | 12 | 27 | 43.61 | 22.5 | 51.58 |
| **SR/SR** | 0 | 0 | NC | NC | NC |
| | 0.5 | 0 | NC | NC | NC |
| | 1 | 15 | 4.39 | 3.62 | 82.52 |
| | 2 | 24 | 25.15 | 11.2 | 44.51 |
| | 4 | 25 | 41.77 | 15.84 | 37.92 |
| | 6 | 25 | 47.17 | 20.4 | 43.24 |
| | 8 | 24 | 45.65 | 20.85 | 45.68 |
| | 10 | 25 | 46.44 | 21.98 | 47.34 |
| | 12 | 25 | 44.08 | 20.09 | 45.57 |

**Table 20. Mean plasma concentrations of the bupropion metabolite erythrohydroxybupropion in subjects receiving bupropion SR in combination with naltrexone IR (IR/SR) or in combination with naltrexone SR (SR/SR).**

| **Treatment** | **Time (hr)** | **N** | **Mean (ng/ml)** | **SD (ng/ml)** | **CV%** |
|---|---|---|---|---|---|
| **IR/SR** | 0 | 0 | NC | NC | NC |
| | 0.5 | 0 | NC | NC | NC |
| | 1 | 4 | 1.24 | 0.15 | 12.42 |
| | 2 | 26 | 3.5 | 1.3 | 37.26 |
| | 4 | 27 | 6.47 | 2.07 | 32.03 |
| | 6 | 27 | 7.7 | 2.42 | 31.38 |
| | 8 | 27 | 7.77 | 2.56 | 32.94 |
| | 10 | 27 | 8.04 | 2.71 | 33.67 |
| | 12 | 27 | 8.05 | 2.4 | 29.8 |
| **SR/SR** | 0 | 0 | NC | NC | NC |
| | 0.5 | 0 | NC | NC | NC |
| | 1 | 2 | 1.64 | 0.43 | 26.22 |
| | 2 | 24 | 3.26 | 1.5 | 46.01 |
| | 4 | 25 | 5.87 | 1.65 | 28.14 |
| | 6 | 25 | 7.07 | 2.17 | 30.75 |
| | 8 | 24 | 7.18 | 2.06 | 28.63 |
| | 10 | 25 | 7.58 | 2.04 | 26.89 |
| | 12 | 25 | 7.51 | 1.92 | 25.51 |

**Table 21** reports the bupropion and bupropion metabolite AUCₗₐₛₜ and the Cₘₐₓ values for the two treatment conditions. These values are similar for both treatments.

**Table 21. Mean pharmacokinetic parameters for bupropion and metabolites on Day 1 in subjects receiving bupropion SR in combination with naltrexone IR (IR/SR) or in combination with naltrexone SR (SR/SR).**

| **Analyte** | | **IR/SR** | | | | **SR/SR** | | | |
|---|---|---|---|---|---|---|---|---|---|
| Bupropion | | **N** | **Mean** | **SD** | **CV%** | **N** | **Mean** | **SD** | **CV%** |
| | AUClast | 23 | 367.6 | 122.4 | 33.3 | 20 | 348.7 | 149.1 | 42.8 |
| | Cmax | 23 | 58.0 | 19.6 | 33.8 | 20 | 57.5 | 23.7 | 41.2 |

| Hydroxybupropion | | | | | | | | | |
|---|---|---|---|---|---|---|---|---|---|
| | AUClast | 27 | 1452.6 | 556.8 | 38.3 | 25 | 1334.0 | 584.5 | 43.8 |
| | Cmax | 27 | 162.3 | 64.4 | 39.7 | 25 | 163.3 | 114.0 | 69.8 |

| Erythrohydroxybupropion | | | | | | | | | |
|---|---|---|---|---|---|---|---|---|---|
| | AUClast | 27 | 74.8 | 22.5 | 30.1 | 25 | 69.6 | 18.5 | 26.7 |
| | Cmax | 27 | 8.5 | 2.7 | 31.5 | 25 | 8.0 | 2.1 | 26.6 |

| Thrcohydroxybupropion | | | | | | | | | |
|---|---|---|---|---|---|---|---|---|---|
| | AUClast | 27 | 456.5 | 209.2 | 45.8 | 25 | 452.0 | 185.0 | 40.9 |
| | Cmax | 27 | 51.2 | 23.0 | 44.8 | 25 | 50.7 | 21.7 | 42.8 |

Adverse event listings as well as dosing logs were used for this analysis. Data was entered into spreadsheets, QA'd, and then summarized for frequency of event, % of subjects reporting various events, time to onset from first dose and day in titration schedule. The incidence of adverse effects obtained using the unprompted method of this example is not directly comparable to the incidence obtained using the methods of Example 4.

As shown in **Table 22,** the percent of subjects reporting any adverse events was 26% and 30% for the SR and IR groups, respectively. The percent of subjects reporting any GI related adverse events was 10% and 16% for the SR and IR groups; similar percentages were observed for subjects reporting any CNS related adverse events. More subjects reported more than one adverse event in the IR group (6/30, 20%), than in the SR group (2/30, 6.6%). Lastly, more subjects reported adverse events that were moderate or greater in severity in the IR group (30.7%) compared with the SR group (16.7%).

**Table 22: Adverse events associated with IR and SR naltrexone with SR bupropion treatment**

| | **Group 1** | **Group 2** |
|---|---|---|
| % Subjects Reporting Any AEs | 26% | 30% |
| Number of Subjects | 8 / 30 | 9 / 30 |
| % Subjects Reporting Any GI related AEs* | 10% | 16% |
| Number of Subjects | 3 / 30 | 5 / 30 |
| % Subjects Reporting Any CNS related AEs** | 10% | 16% |
| Number of Subjects | 3 / 30 | 5 / 30 |
| % Subjects Reporting More Than 1 AE | 6.6% | 20% |
| Number of Subjects | 2 / 30 | 6 / 30 |
| Average Day in Titration Schedule of Onset of AEs | 2 | 4 |
| % AEs Reported as Moderate Severity | 16.7% | 30.7% |
| Distribution of Events | 10 events as mild | 18 events as mild |
| | 2 events as moderate | 8 events as moderate |
| | 0 events as severe | 0 events as severe |

| | | |
|---|---|---|
| * GI related events defined as any report of: nausea, stomach pain, stomach cramps, loss of appetite, diarrhea, emesis, queasiness, dry heaves, increased bowel movements ** CNS related events defined as any report of: headache, dizziness, drowsiness, lightheadedness, sleepiness, lethargy *** Subjects reporting >1 type of GI or CNS adverse event were included in each event frequency | | |

Thus, the group that received the sustained-release naltrexone with bupropion was less likely to experience adverse events than the group that received the immediate-release naltrexone with bupropion. Additionally, the reported adverse events from the group that received the sustained-release naltrexone with bupropion were less severe than those reported from the immediate-release naltrexone group.

4 subjects withdrew consent from the study; none are listed as having not completed the study due to an adverse event. These subjects are not removed from the calculations presented. Three of the 4 subjects reported an adverse event, but none dropped out on the day the event was reported. Three of the subjects were receiving IR, the fourth was receiving SR.

All events (n = 12) in the SR group were reported as mild, with the exception of one instance of euphoria and one instance of stomach cramps (both moderate). Most of the events (n = 26) were reported as mild. There were five GI-related (emesis, stomach pain, nausea) and 3 CNS-related events that were described as moderate (headache, fatigue).

It will be understood by those of skill in the art that numerous and various modifications can be made without departing from the spirit of the present invention. Therefore, it should be clearly understood that the forms of the present invention are illustrative only and are not intended to limit the scope of the present invention.

## Claims

1. A dosage form comprising a sustained-release formulation of naltrexone, or a pharmaceutically acceptable salt thereof and a sustained-release formulation of bupropion or a pharmaceutically acceptable salt thereof, wherein the dosage form is an oral dosage form, wherein the dosage form provides an in vitro release rate of the naltrexone of less than 80% or less than 70% in about 1 hour, of less than 90% or less than 80% in about 2 hours, or of less than 98% in about 4 hours, as determined using United States Pharmacopeia 24th edition (USP 24) Apparatus 2 dissolution test having a spindle rotation speed of 100 rpm and a dissolution medium of water, at 37°C.

2. The dosage form according to claim 1, wherein the dosage form provides an in vitro release rate of the naltrexone of less than 80% or less than 70% in about 1 hour, as determined using United States Pharmacopeia 24th edition (USP 24) Apparatus 2 dissolution test having a spindle rotation speed of 100 rpm and a dissolution medium of water, at 37°C.

3. The dosage form according to claim 1, wherein the dosage form provides an in vitro release rate of the naltrexone of less than 90% or less than 80% in about 2 hours, as determined using United States Pharmacopeia 24th edition (USP 24) Apparatus 2 dissolution test having a spindle rotation speed of 100 rpm and a dissolution medium of water, at 37°C.

4. The dosage form according to claim 1, wherein the dosage form provides an in vitro release rate of the naltrexone of less than 98% in about 4 hours, as determined using United States Pharmacopeia 24th edition (USP 24) Apparatus 2 dissolution test having a spindle rotation speed of 100 rpm and a dissolution medium of water, at 37°C.

5. The dosage form according to any of claims 1-4, comprising a sustained-release carrier selected from one or more of: hydroxypropylmethyl cellulose, polyoxyethylene, polyethylene oxide, polyacrylate, copolymer of acrylate and methacrylate, methacrylate polymer, copolymer of acrylate and methacrylate, copolymer of acrylate and methacrylate with ammonium group, copolymer of maleic anhydride and methyl vinyl ether, hydroxy propyl ethyl cellulose, hydroxy propyl cellulose, hydroxy ethyl cellulose, methyl cellulose, hydroxymethyl methacrylate, maltodextrin, natural gum and xanthan gum.

6. The dosage form according to any of claims 1-5, comprising about 4 mg, about 8 mg, about 12 mg, about 16 mg, about 32 mg, or about 48 mg naltrexone.

7. The dosage form according to any of claims 1-6, comprising about 4 mg, about 8 mg, or about 12 mg of naltrexone and about 90 mg bupropion.

8. The dosage form according to any of claims 1-7, wherein the sustained-release formulation of naltrexone, or a pharmaceutically acceptable salt thereof and the sustained-release formulation of bupropion or a pharmaceutically acceptable salt thereof, are suitable for separate administration.

9. The dosage form according to any of claims 1-7, wherein the dosage form is a unit dose form comprising both the sustained-release formulation of naltrexone, or a pharmaceutically acceptable salt thereof and the sustained-release formulation of bupropion or a pharmaceutically acceptable salt thereof.

10. A dosage form according to any of claims 1-9 for use in treating obesity.

11. The use of an oral dosage form according to any of claims 1-9 in the preparation of a medicament for affecting weight loss or inhibiting weight gain in a patient.

12. The dosage form according to any of claims 1-8 for use according to claim 10, or the use according to claim 11 of a dosage form according to any of claims 1-8, wherein the sustained-release formulation of naltrexone, or a pharmaceutically acceptable salt thereof and the sustained-release formulation of bupropion or a pharmaceutically acceptable salt thereof, are administered separately to the patient.

13. The dosage form for use, or the use according to claim 12, wherein the dosage form is administered as two tablets twice a day, for a daily dosage of about 16 mg, about 32 mg or about 48 mg of naltrexone or a pharmaceutically acceptable salt thereof, and about 360 mg of bupropion or a pharmaceutically acceptable salt thereof.

## Patentansprüche

1. Dosierungsform, umfassend eine Retardformulierung von Naltrexon oder einem pharmazeutisch unbedenklichen Salz davon und eine Retardformulierung von Bupropion oder einem pharmazeutisch unbedenklichen Salz davon, wobei es sich bei der Dosierungsform um eine orale Dosierungsform handelt, wobei die Dosierungsform eine in-vitro-Freisetzungsrate des Naltrexons von weniger als 80 % oder weniger als 70 % in etwa 1 Stunde, von weniger als 90 % oder weniger als 80 % in etwa 2 Stunden oder von weniger als 98 % in etwa 4 Stunden gemäß Bestimmung nach dem Auflösungstest in Vorrichtung 2 der 24. Auflage der US-Pharmakopöe (USP 24) mit einer Spindeldrehgeschwindigkeit von 100 U/min und Wasser als Auflösungsmedium bei 37 °C bereitstellt.

2. Dosierungsform nach Anspruch 1, wobei die Dosierungsform eine in-vitro-Freisetzungsrate des Naltrexons von weniger als 80 % oder weniger als 70 % in etwa 1 Stunde gemäß Bestimmung nach dem Auflösungstest in Vorrichtung 2 der 24. Auflage der US-Pharmakopöe (USP 24) mit einer Spindeldrehgeschwindigkeit von 100 U/min und Wasser als Auflösungsmedium bei 37 °C bereitstellt.

3. Dosierungsform nach Anspruch 1, wobei die Dosierungsform eine in-vitro-Freisetzungsrate des Naltrexons von weniger als 90 % oder weniger als 80 % in etwa 2 Stunden gemäß Bestimmung nach dem Auflösungstest in Vorrichtung 2 der 24. Auflage der US-Pharmakopöe (USP 24) mit einer Spindeldrehgeschwindigkeit von 100 U/min und Wasser als Auflösungsmedium bei 37 °C bereitstellt.

4. Dosierungsform nach Anspruch 1, wobei die Dosierungsform eine in-vitro-Freisetzungsrate des Naltrexons von weniger als 98 % in etwa 4 Stunden gemäß Bestimmung nach dem Auflösungstest in Vorrichtung 2 der 24. Auflage der US-Pharmakopöe (USP 24) mit einer Spindeldrehgeschwindigkeit von 100 U/min und Wasser als Auflösungsmedium bei 37 °C bereitstellt.

5. Dosierungsform nach einem der Ansprüche 1 bis 4, umfassend einen Retardträger, der aus einem oder mehreren von Hydroxypropylmethylcellulose, Polyoxyethylen, Polyethylenoxid, Polyacrylat, Copolymer von Acrylat und Methacrylat, Methacrylat-Polymer, Copolymer von Acrylat und Methacrylat, Copolymer von Acrylat und Methacrylat mit Ammoniumgruppe, Copolymer von Maleinsäureanhydrid und Methylvinylether, Hydroxypropylethylcellulose, Hydroxypropylcellulose, Hydroxyethylcellulose, Methylcellulose, Hydroxymethylmethacrylat, Maltodextrin, Naturgummi und Xanthangummi ausgewählt ist.

6. Dosierungsform nach einem der Ansprüche 1-5, umfassend etwa 4 mg, etwa 8 mg, etwa 12 mg, etwa 16 mg, etwa 32 mg oder etwa 48 mg Naltrexon.

7. Dosierungsform nach einem der Ansprüche 1-6, umfassend etwa 4 mg, etwa 8 mg oder etwa 12 mg Naltrexon und etwa 90 mg Bupropion.

8. Dosierungsform nach einem der Ansprüche 1 bis 7, wobei die Retardformulierung von Naltrexon oder einem pharmazeutisch unbedenklichen Salz davon und die Retardformulierung von Bupropion oder einem pharmazeutisch unbedenklichen Salz davon für die separate Verabreichung geeignet sind.

9. Dosierungsform nach einem der Ansprüche 1-7, wobei es mich bei der Dosierungsform um eine Einzeldosisform handelt, die sowohl die Retardformulierung von Naltrexon oder einem pharmazeutisch unbedenklichen Salz davon als auch die Retardformulierung von Bupropion oder einem pharmazeutisch unbedenklichen Salz davon umfasst.

10. Dosierungsform nach einem der Ansprüche 1-9 zur Verwendung bei der Behandlung von Obesitas.

11. Verwendung einer oralen Dosierungsform nach einem der Ansprüche 1-9 bei der Herstellung eines Medikaments zur Bewirkung von Gewichtsverlust oder Inhibierung von Gewichtszunahme bei einem Patienten.

12. Dosierungsform nach einem der Ansprüche 1-8 zur Verwendung nach Anspruch 10 oder Verwendung nach Anspruch 11 einer Dosierungsform nach einem der Ansprüche 1-8, wobei die Retardformulierung von Naltrexon oder einem pharmazeutisch unbedenklichen Salz davon und die Retardformulierung von Bupropion oder einem pharmazeutisch unbedenklichen Salz davon separat an den Patienten verabreicht werden.

13. Dosierungsform zur Verwendung oder Verwendung nach Anspruch 12, wobei die Dosierungsform für eine Tagesdosierung von etwa 16 mg, etwa 32 mg oder etwa 48 mg Naltrexon oder einem pharmazeutisch unbedenklichen Salz davon und etwa 360 mg Bupropion oder einem pharmazeutisch unbedenklichen Salz davon in Form von zwei Tabletten zweimal täglich verabreicht wird.

## Revendications

1. Forme pharmaceutique comprenant une formulation à libération prolongée de naltrexone, ou d'un sel pharmaceutiquement acceptable de celle-ci, et une formulation à libération prolongée de bupropion, ou d'un sel pharmaceutiquement acceptable de celui-ci, la forme pharmaceutique étant une forme pharmaceutique orale, la forme pharmaceutique assurant une vitesse de libération *in vitro* de la naltrexone, telle que déterminée à l'aide du test de dissolution sur l'appareil 2 selon la 24^{ème} édition de la pharmacopée américaine (USP 24) avec une vitesse de rotation des palettes de 100 tr/min et un milieu de dissolution constitué d'eau à 37 °C, de moins de 80 % ou moins de 70 % en environ 1 heure, de moins de 90 % ou moins de 80 % en environ 2 heures ou de moins de 98 % en environ 4 heures.

2. Forme pharmaceutique selon la revendication 1, la forme pharmaceutique assurant une vitesse de libération *in vitro* de la naltrexone, telle que déterminée à l'aide du test de dissolution sur l'appareil 2 selon la 24^{ème} édition de la pharmacopée américaine (USP 24) avec une vitesse de rotation des palettes de 100 tr/min et un milieu de dissolution constitué d'eau à 37 °C, de moins de 80 % ou moins de 70 % en environ 1 heure.

3. Forme pharmaceutique selon la revendication 1, la forme pharmaceutique assurant une vitesse de libération *in vitro* de la naltrexone, telle que déterminée à l'aide du test de dissolution sur l'appareil 2 selon la 24^{ème} édition de la pharmacopée américaine (USP 24) avec une vitesse de rotation des palettes de 100 tr/min et un milieu de dissolution constitué d'eau à 37 °C, de moins de 90 % ou moins de 80 % en environ 2 heures.

4. Forme pharmaceutique selon la revendication 1, la forme pharmaceutique assurant une vitesse de libération *in vitro* de la naltrexone, telle que déterminée à l'aide du test de dissolution sur l'appareil 2 selon la 24^{ème} édition de la pharmacopée américaine (USP 24) avec une vitesse de rotation des palettes de 100 tr/min et un milieu de dissolution constitué d'eau à 37 °C, de moins de 98 % en environ 4 heures.

5. Forme pharmaceutique selon l'une quelconque des revendications 1-4, comprenant un vecteur de libération prolongée choisi parmi un ou plusieurs de : l'hydroxypropylméthylcellulose, le polyoxyéthylène, le poly(oxyde d'éthylène), un polyacrylate, un copolymère d'acrylate et de méthacrylate, un polymère de méthacrylate, un copolymère d'acrylate et de méthacrylate, un copolymère d'acrylate et de méthacrylate portant un groupe ammonium, un copolymère d'anhydride maléique et d'éther de méthyle et de vinyle, l'hydroxypropyléthylcellulose, l'hydroxypropylcellulose, l'hydroxyéthylcellulose, la méthylcellulose, le méthacrylate d'hydroxyméthyle, la maltodextrine, la gomme naturelle et la gomme xanthane.

6. Forme pharmaceutique selon l'une quelconque des revendications 1-5, comprenant environ 4 mg, environ 8 mg, environ 12 mg, environ 16 mg, environ 32 mg ou environ 48 mg de naltrexone.

7. Forme pharmaceutique selon l'une quelconque des revendications 1-6, comprenant environ 4 mg, environ 8 mg ou environ 12 mg de naltrexone et environ 90 mg de bupropion.

8. Forme pharmaceutique selon l'une quelconque des revendications 1-7, la formulation à libération prolongée de naltrexone, ou d'un sel pharmaceutiquement acceptable de celle-ci, et la formulation à libération prolongée de bupropion, ou d'un sel pharmaceutiquement acceptable de celui-ci, étant appropriées pour une administration séparée.

9. Forme pharmaceutique selon l'une quelconque des revendications 1-7, la forme pharmaceutique étant une forme pharmaceutique unitaire comprenant à la fois la formulation à libération prolongée de naltrexone, ou d'un sel pharmaceutiquement acceptable de celle-ci, et la formulation à libération prolongée de bupropion, ou d'un sel pharmaceutiquement acceptable de celui-ci.

10. Forme pharmaceutique selon l'une quelconque des revendications 1-9 destinée à être utilisée en traitement de l'obésité.

11. Utilisation d'une forme pharmaceutique orale selon l'une quelconque des revendications 1-9 dans la préparation d'un médicament destiné à influer sur la perte de poids ou à inhiber la prise de poids chez un patient.

12. Forme pharmaceutique selon l'une quelconque des revendications 1-8 destinée à être utilisée selon la revendication 10, ou utilisation selon la revendication 11 d'une forme pharmaceutique selon l'une quelconque des revendications 1-8, la formulation à libération prolongée de naltrexone, ou d'un sel pharmaceutiquement acceptable de celle-ci, et la formulation à libération prolongée de bupropion, ou d'un sel pharmaceutiquement acceptable de celui-ci, étant administrées séparément au patient.

13. Forme pharmaceutique destinée à être utilisée ou utilisation selon la revendication 12, la forme pharmaceutique étant administrée sous forme de deux comprimés deux fois par jour, pour une dose quotidienne d'environ 16 mg, d'environ 32 mg ou d'environ 48 mg de naltrexone ou d'un sel pharmaceutiquement acceptable de celle-ci et d'environ 360 mg de bupropion ou d'un sel pharmaceutiquement acceptable de celui-ci.
